# EUROPÄISCHE PATENTSCHRIFT

(11) **EP 4 161 361 B1**
(45) Veröffentlichungstag und Bekanntmachung des Hinweises auf die Patenterteilung: **11.03.2026**
(21) Anmeldenummer: 21733893.8
(22) Anmeldetag: 02.06.2021
(51) Int. Cl.: A61B 5/00, A61N 1/372, A61N 2/00, A61B 5/374

(54) **KÖRPERZUSTANDSABHÄNGIGE TRANKRANIELLE STIMULATION MIT ECHTZEITKOMMUNIKATION ZWISCHEN EINEM AKTIONS- UND EINEM EEG-ERFASSUNGSMODUL**
BODY-CONDITION-DEPENDENT TRANSCRANIAL STIMULATION WITH REAL-TIME COMMUNICATION BETWEEN AN ACTION MODULE AND AN EEG CAPTURE MODULE
STIMULATION TRANSCRANIENNE DÉPENDANT DE L'ÉTAT DU CORPS AVEC COMMUNICATION EN TEMPS RÉEL ENTRE UN MODULE D'ACTION ET UN MODULE DE DÉTECTION D'EEG

(30) Priorität: 03.06.2020 DE 102020114745
(43) Veröffentlichungstag der Anmeldung: 12.04.2023
(73) Patentinhaber: NEUROCARE GROUP AG, 80331 München (DE)
(72) Erfinder: BERKES, Sebastian, 98693 Ilmenau (DE); STEIN, Patrick, 98693 Ilmenau (DE); SCHELLHORN, Klaus, 98693 Ilmenau (DE)
(74) Vertreter: noventive Patentanwaltsgesellschaft mbH
(86) Internationale Anmeldenummer: PCT/EP2021/064780
(87) Internationale Veröffentlichungsnummer: WO 2021/245129

(56) Entgegenhaltungen:
- WO-A2-2018/033591
- US-A1- 2014 330 345
- US-A1- 2018 020 941
- US-A1- 2020 054 888
- US-A1- 2021 124 421
- US-B1- 10 596 371
- US-B1- 12 290 688
- H. N. SURESH ET AL: "Removal OF EMG and ECG artifacts from EEG based on real time recurrent learning algorithm", INTERNATIONAL JOURNAL OF PHYSICAL SCIENCES, vol. 3, no. 5, 1 May 2008 (2008-05-01), US, pages 120 - 125, XP093277805, ISSN: 1992-1950, Retrieved from the Internet <URL:https://academicjournals.org/journal/IJPS/article-abstract/9BE69DD14558> DOI: 10.5897/IJPS.9000484
- JAFARIFARMAND AYSA ET AL: "Real-Time Cardiac Artifact Removal from EEG Using a Hybrid Approach", 2018 INTERNATIONAL CONFERENCE BIOMDLORE, IEEE, 28 June 2018 (2018-06-28), pages 1 - 5, XP033405313, [retrieved on 20180917], DOI: 10.1109/BIOMDLORE.2018.8467207
- ZRENNER BRIGITTE ET AL: "Brain oscillation-synchronized stimulation of the left dorsolateral prefrontal cortex in depression using real-time EEG-triggered TMS", BRAIN STIMULATION, ELSEVIER, AMSTERDAM, NL, vol. 13, no. 1, 12 October 2019 (2019-10-12), pages 197 - 205, XP085926499, ISSN: 1935-861X, [retrieved on 20191012], DOI: 10.1016/J.BRS.2019.10.007
- GATTINGER N ET AL: "flexTMS A Novel Repetitive Transcranial Magnetic Stimulation Device With Freely Programmable Stimulus Currents", IEEE TRANSACTIONS ON BIOMEDICAL ENGINEERING, IEEE, USA, vol. 59, no. 7, 1 July 2012 (2012-07-01), pages 1962 - 1970, XP011490128, ISSN: 0018-9294, DOI: 10.1109/TBME.2012.2195180
- SCHLEGELMILCH F ET AL.: "P 215. A method for online correction of artifacts in EEG signals during transcranial electrical stimulation", CLINICAL NEUROPHYSIOLOGY, vol. 124, no. 10, 1 October 2013 (2013-10-01), XP028703748, ISSN: 1388-2457, DOI: 10.1016/J.CLINPH.2013.04.291
- MEMARIAN SORKHABI MAJID ET AL: "Deep-Brain Transcranial Stimulation: A Novel Approach for High 3-D Resolution", IEEE ACCESS, vol. 5, 28 March 2017 (2017-03-28), pages 3157 - 3171, XP011644195, [retrieved on 20170327], DOI: 10.1109/ACCESS.2017.2672566
- JOACHIM VON ZITZEWITZ ET AL: "A neurorobotic platform for locomotor prosthetic development in rats and mice", JOURNAL OF NEURAL ENGINEERING, INSTITUTE OF PHYSICS PUBLISHING, BRISTOL, GB, vol. 13, no. 2, 10 February 2016 (2016-02-10), pages 26007, XP020300685, ISSN: 1741-2552, [retrieved on 20160210], DOI: 10.1088/1741-2560/13/2/026007
- DAVIDE CILIBERTI ET AL: "Falcon: a highly flexible open-source software for closed-loop neuroscience", JOURNAL OF NEURAL ENGINEERING, INSTITUTE OF PHYSICS PUBLISHING, BRISTOL, GB, vol. 14, no. 4, 21 June 2017 (2017-06-21), pages 45004, XP020318481, ISSN: 1741-2552, [retrieved on 20170621], DOI: 10.1088/1741-2552/AA7526
- PATEL YOGI A. ET AL: "Hard real-time closed-loop electrophysiology with the Real-Time eXperiment Interface (RTXI)", PLOS COMPUTATIONAL BIOLOGY, vol. 13, no. 5, 30 January 2017 (2017-01-30), US, pages e1005430, XP055843227, ISSN: 1553-734X, DOI: 10.1371/journal.pcbi.1005430
- ROH TAEHWAN ET AL: "A Wearable Neuro-Feedback System With EEG-Based Mental Status Monitoring and Transcranial Electrical Stimulation", IEEE TRANSACTIONS ON BIOMEDICAL CIRCUITS AND SYSTEMS, IEEE, US, vol. 8, no. 6, 1 December 2014 (2014-12-01), pages 755 - 764, XP011570654, ISSN: 1932-4545, [retrieved on 20150115], DOI: 10.1109/TBCAS.2014.2384017

## Beschreibung

Die Offenbarung betrifft ein System zur körperzustandsabhängigen Stimulation mit Echtzeitkommunikation zwischen einem Aktions- und einem Erfassungsmodul. Dabei wird biologisches Gewebe stimuliert, bei gleichzeitiger Erfassung von Signalen biologischen Ursprungs. Die Anwendung dieses Verfahrens und der Anordnung betrifft vorwiegend, aber nicht ausschließlich alle Bereiche der Medizin, in denen Biosignale für eine körperzustandsabhängige Stimulation genutzt werden.

### Stand der Technik

Eine Vorrichtung zur transkraniellen Stimulation ist von PATEL YOGI A. ET AL: "Hard real-time closed-loop electrophysiology with the Real-Time experiment Interface (RTXI)",PLOS COMPUTATIONAL BIOLOGY, Bd. 13, Nr. 5, Januar 2017, bekannt. 1

Es gibt medizinische Behandlungsmethoden, wie in der WO2017099603 (A1) beschrieben, bei denen eine fokale Neuromodulationstechnik angewandt wird, für die die korrekte Position auf der Hirnrinde bestimmt werden muss, die geeignet ist, psychische oder neurologische Probleme besser zu behandeln. Diese Behandlung kann mittels Transkranieller Magnetstimulation (TMS)) bewirkt werden, wobei gleichzeitig eine Messung des Herzschlags erfolgt. Wenn eine systematische Änderung in Form einer Herzratenreduktion (Änderung der Herzratenvariabilität) erkennbar ist, gilt die Position als lokalisiert. Die Anzeige der Herzrate erfolgt in unmittelbarer Reaktion auf wiederholte Impulsfolgen der repetitiven TMS.

Für den Nachweis dieses Reiz-Reaktionsmusters ist eine unmittelbare Messung notwendig, um die Reaktion der Stimulation korrekt zuordnen zu können und damit auch die richtige Schlussfolgerung über die medizinisch geeigneten Positionen des Stimulationsortes treffen zu können.

Nicht offenbart ist in der Schrift jedoch, wie die unmittelbare Reaktion, bzw. die schnelle Rückkopplung technisch umgesetzt werden kann.

Üblicherweise werden die Module für die Aktion (Stimulation mit TMS) und Erfassung (Messung des EKG) mit einem handelsüblichen Bussystem miteinander verbunden. Damit kann jedoch die unmittelbare Kommunikation und darüber hinaus die Kommunikation nicht in Echtzeit gewährleistet werden.

Unabhängig davon existieren Ethernet-Übertrager, wie in der Zeitschrift "Elektronikpraxis" Nr. 8 vom 16.04.2019 auf S.19 beschrieben ist, die eine Schutzvorrichtung, z. B. mittels galvanischer Trennung, bilden.

Ausgehend von bekannten Systemen zur körperzustandsabhängigen Stimulation stellt sich an die Erfindung die Aufgabe, ein System anzugeben, mit dem vergleichsweise zuverlässig und sicher eine körperzustandsabhängige Stimulation durchgeführt werden kann.

Die Erfindung löst diese Aufgabe durch ein System gemäß Anspruch 1. Bevorzugte Ausführungsformen sind in den abhängigen Patentansprüchen erläutert.

Biodaten sind Daten, die durch Messungen biologischer Messgrößen entstehen. Biologische Signale sind ebenfalls Biodaten, bzw. im Speziellen ein Datenstrom verschiedener zeitkontinuierlicher oder zeitdiskreter Messungen mit Aussagekraft über den Zustand von Gewebe oder eines Organs. Solche Daten oder Signale liefern dabei Informationen über die Funktion von Organen innerhalb eines Organismus (z. B. EKG, EEG, EMG, EOG, ERG, PPT, Atmung, MKG, MEG, BD, SpO2) mit deren Hilfe sich auch eine Auswirkung einer vorangegangenen (oder quasi-simultanen) Stimulation auf den Körper messen lässt. Voraussetzung für die Güte einer (der Messung folgenden) körperzustandsabhängigen Stimulation ist neben adäquater Signalverarbeitung, Merkmalsextraktion und gezielter Beeinflussung des menschlichen Körpers eine artefakt- und störungsfreie Stimulation, die durch das erfindungsgemäße System aufgrund der Echtzeitanforderung (Unmittelbarkeit) für die Kommunikation zwischen den Erfassungs- und Aktionsmodulen des Systems gewährleistet ist.

In Abhängigkeit von einer therapeutischen Vorgabe werden Biosignale von Körperstellen abgeleitet. Das kann z. B. sowohl am Kopf (EEG) aber auch am Körper (z. B. EMG, EKG, BP, SpO2) erfolgen.

Während einer typischen Behandlung werden die Biosignale kontinuierlich vor, während bzw. nach einer medizinischen Intervention von gleichen oder aber auch unterschiedlichen Sensoren aufgenommen. Dabei ist der zeitliche Abstand von unmittelbar, also innerhalb von Mikro- bis Millisekunden, bis zu Minuten oder Stunden möglich.

Aus Signalen, die beispielsweise verstärkt und für eine digitale Signalverarbeitung aufbereitet wurden, können artefaktfreie Signale und diejenigen Informationen gewonnen werden, die für eine therapeutische Intervention von Bedeutung sind. Merkmale sind hierbei z.B. Änderungen der momentanen Bandleistungen, Amplitude, Frequenz bzw. Phase im Elektroenzephalogramm (EEG), individuelle Alpha-Frequenz im EEG, das systolische Intervall des kontinuierlichen Blutdrucks (BP oder BD abgekürzt), der Effektivwert des EMG, das Muster des photoplethysmografischen Signals, die Sauerstoffsättigung im Blut (SpO2) und weitere, bzw. daraus kombinierte Größen. Sensoren zur Erfassung/Messung der Biodaten, Biosignale oder Signale biologischen Ursprungs befinden sich bevorzugt im Erfassungsmodul, auch Erfassungseinheit genannt. Ein solches Modul, hierin auch als Funktionsmodul bezeichnet, ist mit einer Schnittstelle zur Anbindung an einen Echtzeit-Bus, insbesondere einen EtherCAT-Bus, ausgebildet.

Dazu weist das Funktionsmodul einen Schnittstellenabschnitt auf, der die Schnittstelle zu einem extern anschließbaren oder einem in das Funktionsmodul integrierten Gerät, oder einer nachgeschalteten Einrichtung aufweist. Des Weiteren enthält ein derartiges Funktionsmodul einen Verbindungs- und Steuerabschnitt. Der Verbindungs- und Steuerabschnitt weist dabei vorzugsweise einen Mikrocontroller als Steuereinheit für das Funktionsmodul, und einen Kontaktabschnitt, der zur Anbindung an den Echtzeit-Bus der Vorrichtung ausgebildet ist, auf.

Die Signalverarbeitung oder Auswertung der Biodaten oder Biosignale kann entweder bereits im Erfassungsmodul oder erst im Aktionsmodul stattfinden oder einem separaten Auswertemodul, auch Signalverarbeitungsmodul bzw. Mastermodul genannt, das über die Kommunikationsverbindung mit dem Aktions- und Erfassungsmodul kommuniziert.

Das Aktionsmodul umfasst z.B. Aktoren, die auf das Gewebe bzw. die Organe bzw. Körper im Allgemeinen einwirken können. Das Aktionsmodul kann auch lediglich die Schnittstelle zu derartigen Aktoren bereitstellen, wobei die eigentlichen Aktoren dann wiederum als externe Komponenten an das Mastermodul anschließbar sind.

Grundsätzlich kann das biologische Organ oder Gewebe z.B. mittels elektrischer, magnetischer, elektro-magnetischer, mechanischer, pneumatischer bzw. hydraulischer Aktoren beeinflusst werden. Auf die Körperstellen werden elektrische Ströme durch Elektroden, magnetische Felder durch Spulen, mechanische Kräfte durch Aktoren, Gase bzw. Flüssigkeiten durch Schläuche bzw. Leitungen ein- bzw. aufgebracht.

Für eine Stimulation, die abhängig vom Körperzustand ist, wird z.B. eine Rückkopplung benötigt, die die Biodaten oder Biosignale Einfluss nehmen lässt auf die Art und Weise, wie die Aktoren angesteuert werden. Eine entsprechende Steuerung oder ein (ggf. geschlossener) Regelkreis bieten sich hier an.

Hierbei können folgende Aspekte berücksichtigt werden:
- Die Aktoren können bevorzugt für eine körperzustandsabhängige Stimulation schnell auf Information über den Körperzustand (die Biodaten oder Biosignale) reagieren;
- die Aktoren geben bevorzugt nur die Signale ab, die für die Beeinflussung des menschlichen Körpers / der Organe von Bedeutung sind bzw. gewünscht werden;
- die Signalpegel der Stimulation liegen bevorzugt als digitale Signale zeit- bzw. wertediskret vor, die nach Wandlung und Verstärkung (Anpassung) an den Aktor in Form von mechanischer bzw. elektromagnetischer Energie abgegeben werden;
- die Signalpegel zur Erfassung liegen nach Wandlung z.B. im Bereich von Nano- bis Millivolt innerhalb eines Frequenzbandes von Null bis einigen Kilohertz vor;
- im genutzten (z. B. dem für die Merkmalsextraktion aus dem EEG herangezogenen) Frequenzband treten durch die Stimulation und die Umgebung z.B. starke Störsignale auf; diese Störsignale können erfindungsgemäß berücksichtigt und, je nach Bedarf, herausgefiltert werden;
- die zu untersuchenden Signalquellen, z. B. elektrophysiologischen Ursprungs, sind bevorzugt hochohmig;
- die physikalischen Eigenschaften z. B. der Stimulations- und Erfassungselektroden verändern sich beispielsweise mit der Zeit (z. B. durch Änderung der Elektrodenübergangsimpedanzen, Elektrodenspannung, Offsetpotentiale, Andruckbedingungen oder durch Bewegungsartefakte).

Es sind Stimulations- und Signalerfassungssysteme bekannt, die diese Probleme, bei sorgfältiger Wahl der Ableitmethodik, entsprechender Verstärker-, Auswerte- und Stimulationstechnik, teilweise beherrschen. Jedoch sind hochwertige kommerzielle Stimulations- und Polygraphiesysteme zur körperzustandsabhängigen Stimulation und gleichzeitigen Aufzeichnung und Auswertung von Biosignalen unterschiedlichen physiologischen Ursprungs sehr kostenintensiv und meist nur für den stationären Einsatz vorgesehen.

Im Folgenden wird als Beispiel zur körperzustandsabhängigen Stimulation auf Grundlage der Erfassung von Signalen biologischen Ursprungs die gegenwärtige Vorgehensweise mittels Verwendung von Aktoren und Sensoren erläutert.

Stromstimulatoren für die kraniale Elektrotherapie werden in der heutigen Zeit als Mikrocontroller angesteuerte Konstantstromquellen realisiert. Dies erlaubt eine elektrische Stimulation mit beliebigen Stromformen. Um das analoge Stromsignal zu erzeugen, werden die Stromformen vom Mikrocontrollerprogramm digital bereitgestellt. Die notwendige Digital-Analog-Wandlung kann aufgrund einer zu geringen Abtastung und eines zu kleinen Dynamikbereichs zu systematischen Fehlern im analogen Stimulationsstromsignal führen und unerwünschte Frequenzlinien im EEG-Spektrum zeigen.

Die Messung des EEG während der Stromstimulation stellt ebenfalls hohe Anforderungen an die EEG-Ableittechnik und Ableitung des EEG. Anforderungen bei EEG Messungen sind beispielsweise:
- Vermeidung der Sättigung des Verstärkers durch ausreichende Amplitudenauflösung;
- Vermeidung von Netzinterferenzen durch Verwendung batteriebetriebener Erfassungseinheiten;
- Vermeidung des störenden kapazitiven Einflusses der äußeren Hautschicht auf das Signal durch sorgfältiges Präparieren der Haut.

In anderen Anwendungen, beispielsweise in EKG Messungen, können dieselben Anforderungen oder ein teil dieser Anforderungen ebenfalls gelten.

Gegenwärtige Anordnungen weisen in der Regel wenigstens einen der folgenden Nachteile auf:
- Langsamkeit der Verarbeitung, da kein Echtzeitsystem für eine körperzustandsabhängige Stimulation vorliegt, wenn gleichzeitig Artefaktkorrektur und Merkmalsextraktion durchgeführt werden müssen;
- Instabilität, wenn kleinere, insbesondere nicht konstante, Verzögerungen für eine feedbackgesteuerte / regelungsgekoppelte Anwendung auftreten;
- Komplexität, wenn erhebliche Tests oder Anpassungen nach Änderungen am Aufbau oder am Betriebssystem erforderlich sind.

Die Erfindung stellt in einer Ausführungsform ein System zur körperzustands-abhängigen Stimulation mit zwei Funktionsmodulen, beispielsweise einem Aktionsmodul zur Stimulation von Gewebe und einem Erfassungsmodul zur Ableitung bzw. Messung von Biodaten oder Biosignalen, bereit, bei dem die Kommunikation zwischen beiden Modulen über eine Kommunikationsverbindung erfolgt, die harte oder zumindest feste Echtzeitanforderungen erfüllt.

Das Aktionsmodul und das Erfassungsmodul können z.B. Prozessormittel zur Datenverarbeitung wie z.B. eine "Central Processing Unit" (CPU) aufweisen. Sie können zudem mit elektronischen Datenspeichern zur Datenspeicherung wie z.B. einem Flashspeicher oder einer "Solid-State-Disk" (SSD) ausgestattet sein.

Beide Module können in einem einzigen Gehäuse zusammengefasst sein und Komponenten eines einzigen Geräts ausbilden. Es kann jedoch auch vorgesehen sein, z.B. das Aktionsmodul und das Erfassungsmodul separat und/oder mobil auszubilden, um eine Anbringung an einem Patienten zu erleichtern.

Die mittels des Erfassungsmoduls erfassten Biodaten oder Biosignale können mittels einer Auswerteanordnung genauer analysiert werden. Diese Auswerteanordnung kann lokal bei dem Erfassungsmodul, z.B. als Bauteil in einem Gerät oder entfernt, z.B. an einem Computerarbeitsplatz, eingerichtet sein. Eine Kommunikationsverbindung zwischen dem Erfassungsmodul und der Auswerteanordnung kann kabelgestützt per Lichtwellenleiter, Kupferkabel oder unmittelbar durch gedruckte Leiterbahnen in einer entsprechenden integrierten Vorrichtung erfolgen; sie kann jedoch auch per Funk über z.B. einen der Standards GPRS, 3G, 4G (LTE), 5G, 6G erfolgen, soweit die notwendigen Zeitanforderungen, insbesondere Echtzeitanforderungen, eingehalten werden können.

Die Analyse und Ausgabe der Daten, beispielsweise auf einer integrierten Anzeigevorrichtung, kann, insbesondere in einem Fall, in dem nur eine Messung und nicht eine stimulationsabhängige Feedback-Modulation notwendig ist, geringere Aktualität als die Kommunikation zwischen dem Mastermodul und den Funktionsmodulen aufweisen, so dass beispielswiese in einer Ausgestaltung der Erfindung auch eine nicht-Echtzeitfähige Datenübertragung mit Latenzzeiten über 100ms ausreicht.

Geeignete Protokolle zur Datenübermittlung an externe Auswerteeinheiten können z.B. TCP/IP für eine Nutzung des Internets oder Ethernet sein. Bei einer Nutzung des Internets zur Datenübermittlung werden patientenbezogene Daten bevorzugt kryptographisch verschlüsselt übermittelt.

Eine entfernte Auswerteanordnung ermöglicht es im Sinne einer telemedizinischen Anwendung beispielsweise, dass ein Experte für die Auswertung von Biodaten oder Biosignalen nicht vor Ort bei der Datenerfassung am Patienten präsent sein muss, sondern vielmehr remote, also örtlich von dem Patienten getrennt, Echtzeitmessungen begutachten kann. Damit ist die Erfindung noch universeller einsetzbar.

Die Auswerteanordnung kann beispielsweise als ein Server mit CPU und Datenspeicher eingerichtet sein. Die Analyse der Messdaten, die vom Erfassungsmodul übermittelt werden, kann per Software erfolgen. Die Auswerteeinrichtung kann jedoch auch als reine Softwarekomponente in einer Cloud angeboten werden. Eine Cloud ist ein Computernetzwerk mit vielen Datenprozessor- und Datenspeicher-Ressourcen, die zentral gesteuert und skalierbar, d.h. je nach Bedarf, einer Anwendung zur Verfügung gestellt werden kann. Diese Ausführungsform hat den Vorteil, dass viele unterschiedliche Datensätze unterschiedlicher Patienten (ggf. anonymisiert) zusammengeführt und verglichen werden können.

Die Auswerteanordnung kann Software für maschinelles Lernen, d.h. z.B. für ein neuronales Netz und sog. "Deep Learning" aufweisen, um aus den erfassten Daten eines Patienten oder vieler Patienten (insbesondere bei einer Cloud Lösung) Muster zu erkennen. Solche Muster können sich beispielsweise im Falle der Erfassung eines Elektroenzephalogramms (EEG), d.h. von auf der Kopfhaut eines Patienten mit Elektroden ableitbaren elektrischen Strömen, auf charakteristische Wellenformen im EEG als Resultat der Stimulation durch das Aktionsmodul beziehen.

Mit anderen Worten gewährleistet das erfindungsgemäße System, dass die Echtzeitanforderung (Unmittelbarkeit) für die Kommunikation zwischen den Modulen des Systems erfüllt ist. Stimulationen des menschlichen Körpers können aufgrund der erfindungsgemäß gleichzeitigen Erfassung von Biodaten und den körperzustandsabhängigen Stimulationen intensiviert oder zielgerichteter erfolgen. Zudem kann ermöglicht werden, dass, beispielsweise mittels eines Steuerungsmoduls, ein Triggerimpuls gemäß vorbestimmter Parameter ausgelöst werden kann.

Das Erfassungsmodul muss in der Lage sein, schnell und in Echtzeit das EEG-Signal während der Stromstimulation von den Artefakten zu befreien. Hierzu bedarf es neben der Technik zur Erfassung von Signalen mit hohem Dynamikbereichen (1 µV bis 250 mV) auch Kenntnisse über die Entstehung und Interpretation der Artefakte durch Stromstimulation und deren Vermeidung bzw. Beseitigung. Dazu muss auch eine Information über das Verhalten des Aktionsmoduls vorliegen, sei es da sich dieses prädiktiv verhält oder eine (mittelbare) Kommunikation vom Aktionsmodul zur Erfassungseinheit stattfindet. Ein derartiger Informationsfluss zwischen den Modulen würde wiederum über den Echtzeit-Bus erfolgen, so dass eine Information von dem Aktionsmodul in ein Datenpaket des Mastermoduls hochgeladen wird, das dann in einem Folgepaket, in der Regel eine Millisekunde später bei der erfindungsgemäß bevorzugten Taktung des integrierten Stimulations- und Messsystems (MIS), dem Erfassungsmodul zum Download aus dem nächsten Datenpaket bereitgestellt wird.

In besonders bevorzugten Ausführungen der Erfindung erfolgt die Datenverarbeitung, Entfernung von Artefakten und ähnliches in dem Mastermodul. Die Funktionsmodule sind dabei beispielsweise dazu vorgesehen, die Daten zu erfassen und an das Mastermodul zu senden, Aktoren entsprechend der Befehle des Mastermoduls anzusteuern oder Triggersignale zur Steuerung externer Geräte auszulösen. Auf diese Weise kann beispielsweise eine Echtzeit-Ausführung der Befehle des Mastermoduls verbessert werden, da keine komplexen Berechnungsschritte von den Funktionsmodulen durchzuführen sind.

Für die kombinierte Messung des EEG während der Stimulation (z. B. durch tES) können ferner Filteralgorithmen notwendig oder vorteilhaft sein, die in der durch das erfindungsgemäße System bereitgestellten Echtzeit angewendet werden müssen und kaum Verzögerung zwischen Auftreten des Ereignisses und extrahiertem Merkmal dulden. Diese Algorithmen müssen sowohl in Bezug auf ihre Geschwindigkeit (Ziel: kleine Verzögerung) und Störunterdrückung (Ziel: gute Signalrekonstruktion) den Anforderungen genügen. Dynamische Regressionsmodelle kommen hierbei nicht zur Anwendung, da sie ein Template benötigen und deshalb einer Verzögerung von mehreren Sekunden unterliegen können. Ein solches Template ist ein Muster, das allgemein die Artefakte beschreibt und welches aus Messungen gebildet wird. Da das Biosignal niemals vorhergesagt werden kann, muss der Einfluss des Artefakts auf das Biosignal abgeschätzt werden.

Die Entfernung von Artefakten aus den Messsignalen, insbesondere hervorgerufen durch externe Einflüsse aus der Umgebung oder auch durch den oder die Stimulationstrigger, ist dabei unter anderem abhängig von den Modalitäten der Neuromodulation, beispielsweise ob es sich um TMS oder tES Messverfahren handelt. Dabei kann es sich je nach Anwendungsfall beispielsweise um rekursive Methoden zur Filterung handeln, um FIR-Filterung und ähnliches, wenn die Messung während einer Neuromodulation erfolgen soll.

In einer bevorzugten Ausführungsform des erfindungsgemäßen Systems weist das Erfassungsmodul eine Filtereinrichtung auf. Die Filtereinrichtung ist z.B. als ein Bandpassfilter ausgebildet, der Störsignale in den erfassten Biodaten oder Biosignalen herausfiltert. Störsignale weisen z.B. Frequenzen auf, die z.B. außerhalb eines vorher festgelegten Frequenzintervalls liegen. Ein Frequenzintervall weist einen oberen und einen unteren Schwellenwert für die Amplitude auf.

Störsignale weisen ferner z.B. Amplituden auf, die z.B. außerhalb eines vorher festgelegten Amplitudenintervalls liegen. Ein Amplitudenintervall weist einen oberen und einen unteren Schwellenwert für die Amplitude auf.

Die herausgefilterten Störsignale bleiben anschließend für weitere Analysen unberücksichtigt, was die Qualität nachgelagerter Analysen verbessert.

Artefakte der Stimulation durch das Aktionsmodul können ebenfalls als Störsignale detektierbar sein und herausgefiltert werden. Da bei den Artefakten aufgrund der Kenntnis der vom Aktionsmodul abgegebenen Stimuli grundsätzlich ein zeitlicher Verlauf der Artefakte bekannt ist, können diese in einer Weiterbildung der Erfindung in Echtzeit herausgefiltert werden. Beispielsweise könnte bei einer transkraniellen Stimulation mit elektromagnetischen Impulsen die eingestrahlte Impulsfolge aus den erfassten Biosignalen oder Biodaten herausgefiltert oder herausgerechnet werden. Die Beeinflussung von z.B. Ableitungselektroden am Kopf eines Patienten durch die eingesetzte Transkranielle Stimulation kann sich z.B. durch direkte Induktion ergeben, wenn in diesem Fall das Aktionsmodul und das Erfassungsmodul nah beieinander am Kopf eines Patienten angeordnet sind.

Andere Arten von Artefakten, die erkannt und herausgefiltert werden können, umfassen z.B. Störungen durch die elektrische Aktivität des Herzens. Wird gleichzeitig zum EEG auch ein Elektrokardiogramm (EKG) aufgenommen, so können diese Artefakte ebenfalls herausgerechnet werden.

In vorteilhaften Weiterbildungen der Erfindung kann die Echtzeitfähigkeit derart optimiert sein, beispielsweise auf eine Datenpaketrate von 1000Hz, also ein Datenpaket pro Millisekunde, dass keine Prädiktion des Einflusses von Messungen oder Stimulationen erfolgen muss, da die hohe zeitliche Auflösung und der genaue Zeitpunkt der Messung bzw. der Stimulation unmittelbar die Berücksichtigung der Artefakte ermöglichen kann.

Zu den Stimulationstechniken gehört die TMS (Transkranielle Magnetstimulation), bei der es sich um eine nichtinvasive Technik der Neuromodulation handelt, die einen direkten Einfluss auf die Hirnfunktion hat. Es werden kurze magnetische Pulse auf dem Kopf eines Patienten abgesetzt, mit dem Ziel elektrische Ströme in den darunter liegenden Neuronen zu induzieren.

Ebenso können andere Elektrostimulationstechniken wie nTMS (navigierte Transkranielle Magnetstimulation), tDCS (transcranial direct current stimulation - Gleichstromstimulation), tACS (transcranial alternating current stimulation - Wechselstromstimulation), tRNS (transcranial random noise stimulation - Rauschstimulation), DBS (deep brain stimulation - Tiefenhirnstimulation), FES (Funktionelle Elektrostimulation), Ultraschall, am Kopf aber auch in der Peripherie (Arm, Bein, Brust, Hals) etc. eingesetzt werden.

Beispiele für zukünftige Anwendungen einer individuellen, patientenspezifischen Anpassung der Hirn-/Neurostimulation mittels elektrischer Ströme (tES) an die Hirnaktivitätsmuster (EEG) können die im Folgenden erläuterten bevorzugten Ausführungsformen der Erfindung sein:
In einer bevorzugten Ausführungsform des erfindungsgemäßen Systems ist das Erfassungsmodul zur Ableitung bzw. Messung eines EEG ausgebildet. Dies ist ein Vorteil, weil mittels eines EEG die Gehirnaktivität ermittelt und beispielsweise basierend darauf eine neuronale Stimulation erfolgen kann. Dies wird typischerweise Neurofeedback genannt.

Die Messung des EEG kann mittels einer Elektrodenanordnung zur Erfassung von Gehirnströmen erfolgen. Die Elektrodenanordnung kann dafür eine Vielzahl, bevorzugt mehr als 10, Elektroden aufweisen, die analog mit einem Analog-Digital-Wandler verbunden sind. Die Elektroden können beispielsweise derart an einer Haube angebracht sein, dass verschiedenste Hirnareale auf elektrische Aktivität überwacht werden können. Die Elektroden können jeweils einen Aktuator zum Anpressen auf den Kopf eines Patienten aufweisen. Der Aktuator kann elektromechanisch angesteuert werden. Bevorzugt ist jeder Aktuator mit einem fluiddichten Schlauch mit einem Kompressor verbunden. Durch den Kompressor kann Überdruck in einem Fluid, z.B. einem Gas wie Luft, erzeugt werden, was zur Ausdehnung des Aktuators führt und die Elektrode anpresst. Der Aktuator kann dafür jeweils einen Druckluftkolben oder ein mit Luft befüllbares Kunststoffkissen aufweisen.

Der Vorteil einer pneumatischen Anpressung mit Druckluft besteht darin, dass jeweils eine besonders gleichmäßige Anpressung aller Elektroden der Haube erreicht wird.

Das Aktionsmodul ist für eine transkranielle Stimulation von Gewebe ausgebildet. Der Begriff transkraniell bezeichnet in diesem Zusammenhang das Einwirken durch den Schädelknochen. Das Gewebe, auf das eingewirkt wird, ist beispielsweise das Gehirn eines Patienten.

In einer bevorzugten Weiterbildung der vorgenannten Ausführungsform ist das Aktionsmodul ausgebildet, einen Hirnrhythmus von außen mittels einer transkraniellen Wechselstromstimulation (tACS) einzuprägen.

In einer weiteren bevorzugten Weiterbildung der vorgenannten Ausführungsform ist das Aktionsmodul ausgebildet, eine phasenbezogene transkranielle Magnetstimulation zur gezielten Hemmung oder Erregung kortikospinaler Bahnen auszulösen.

In einer weiteren bevorzugten Ausführungsform des erfindungsgemäßen Systems ist das Erfassungsmodul ausgebildet, eine Messung der individuellen EEG-Alpha-Peak-Frequenz (iAPF) durchzuführen. Mit der gemessenen iAPF kann beispielsweise eine repetitive Ansteuerung des Aktionsmoduls für wissenschaftliche Studien bzgl. der Behandlung von Menschen mit Depressionen erfolgen. Das Aktionsmodul kann in diesem Zusammenhang eine Stimulation mittels transkranieller Magnetstimulation durchführen.

Ferner können weitere Anwendungen eingesetzt werden:
- Erfassung des EEG und phasenbezogenen TMS für wissenschaftliche Studien bzgl. der Behandlung von Menschen mit Depressionen;
- Entwicklung, Implementierung und Evaluierung von genauen bzw. schnellen Techniken für die Korrektur von Artefakten (tES) im abgeleiteten Biosignal (EEG);
- Entwicklung und Realisierung von selbstkalibrierenden, rauscharmen und in Echtzeit ansteuerbaren Stromquellen für die tES;
- Implementierung und Evaluierung von Techniken zur phasengenauen Detektion von Ereignissen im EEG;
- Entwicklung und Realisierung einer generischen Plattform für ein integriertes Stimulations- und Messsystem (MIS) zur kombinierten EEG-Messung und Multikanal-WechselstromStimulation.

Der zentrale Aspekt der Erfindung betrifft die Kommunikation zwischen den Erfassungs- und Aktionsmodulen über eine Kommunikationsverbindung (verdrahtet oder drahtlos), die harte oder zumindest feste Echtzeitanforderungen erfüllt. Da biologische Vorgänge auch eine zeitliche Komponente beinhalten, kann eine Stimulation nur dann sinnvoll adaptiv ausgeführt werden, wenn die der Adaption zugrundeliegenden Daten rechtzeitig zur Verfügung stehen (feste Echtzeitanforderung). Dazu muss einerseits die Messung innerhalb von gewissen Zeitschranken nach der Stimulation durchgeführt werden, weiterhin innerhalb einer anderen Zeitschranke die Messergebnisse ausgewertet und dem Aktionsmodul für die Folgestimulation zugeführt werden.

Eine harte Echtzeitanforderung ist wie folgt definiert. Eine Überschreitung der Antwortzeit wird als ein Versagen gewertet. Nach einer exakten Zeiterfassung der bereitzustellenden Anwendung sind Berechnungen gemäß der Theorie der Echtzeitsysteme notwendig. Echtzeitsysteme liefern das korrekte Ergebnis immer innerhalb der vorgegebenen Zeitschranken. Auf diese Eigenschaft kann man sich beim Einsatz eines harten Echtzeitsystems verlassen. Eine feste Echtzeitanforderung ist dagegen wie folgt definiert. Bei festen Echtzeitanforderungen droht kein unmittelbarer Schaden. Nach Ablauf der Zeitanforderungen ist das Ergebnis der Berechnung jedoch nutzlos und kann verworfen werden.

Für die Gesamtheit der Messungen kann auf eine feste Echtzeitanforderung zurückgegriffen werden, bei der einzelne Messungen, die die harte Echtzeitanforderung nicht erfüllen, für die zur Ausgabe bestimmten Analyse verworfen, bzw. nicht berücksichtigt werden.

Ein Verwerfen von Messungen ist dabei lediglich für die Datenauswertung relevant. Die Sample an sich bleiben dabei erhalten und das closed-loop Verfahren wird nicht unterbrochen.

Eine Echtzeitmessung soll die Möglichkeit einer sofortigen Auswertung bzw. Betrachtung der Signale ermöglichen. In einer Ausführungsform zur Messung eines EEG sind die Komponenten dabei bis zu einer Frequenz von 600 Hz ausgelegt. Um solche Frequenzen detektieren zu können, müssen die Anforderungen an eine Abtastung nach Shannon gewährleistet sein.

Insbesondere bei Busverbindungen, d. h. mit mehr als 2 Teilnehmern, kann die Erfüllung harter Echtzeitbedingungen bei Kommunikationsverbindungen eine Herausforderung sein, z. B. weil es zu Kollisionen beim Senden durch Teilnehmer kommen kann. Hierfür würden sich klassischerweise Zeitscheibenverfahren oder Polling anbieten.

Ein verbreitetes und daher auch erprobtes und günstiges Bussystem ist das Ethernet, das aufgrund seines CSMA/CD mit Kollisionen und Zeitverzögerungen rechnet und für sich betrachtet nicht echtzeitfähig ist.

Es gibt Erweiterungen des Ethernetstandards, wie Standards aus dem Bereich des Timesensitive Networkings (TSN), die Echtzeitfähigkeit herstellen. Dafür kann weitverbreitete Standardhardware verwendet werden, da der TSN-Anteil auf einer höheren Schicht des Protokolls (ISO/OSI-Modell) agiert.

Alternativ gibt es spezialisierte Systeme am Markt, die für hohe Geschwindigkeiten und Echtzeit optimiert sind, wie z. B. Infiniband, was bei Supercomputern zum Einsatz kommt und eine sehr niedrige Latenz, besser als bei TSN, ermöglicht.

Vorgeschlagen wird erfindungsgemäß die Verwendung eines EtherCAT Bussystems als Kommunikationsverbindung für die Erfassungs- und Aktionsmodule.

Das Bussystem EtherCAT, bei dem es sich um einen internationalen IEC-Standard handelt, gilt als der "Ethernet-Feldbus", weil er die Vorteile von Ethernet mit der Einfachheit der klassischen Feldbussysteme kombiniert und die Komplexität von IT-Technologien vermeidet.

EtherCAT umgeht die Nachteile von Ethernet durch das besonders performante Funktionsprinzip: Ein Frame reicht in der Regel aus, um in allen Teilnehmern die Ausgangsinformationen zu aktualisieren und mit dem gleichen Frame die Eingangsinformationen für die Steuerung einzulesen. Das vom EtherCAT-Master ausgesandte Telegramm durchläuft alle Teilnehmer. Jeder EtherCAT-Slave liest "on the fly" die an ihn adressierten Ausgangsdaten und legt seine Eingangsdaten in den weitergeleiteten Frame. Das Telegramm wird nur durch Hardware-Durchlaufzeiten verzögert. Der letzte Teilnehmer eines Segments (oder Abzweigs) erkennt einen offenen Port und sendet das Telegramm zum Master zurück - hierbei wird die Vollduplex-Eigenschaft der Ethernet-Physik ausgenutzt. Vorteilhafterweise sind alle angeschlossenen Module (Slaves) physisch unmittelbar mit dem Master über den Echtzeit-Bus verbunden (MAC-to-MAC-Kommunikation). So ist eine direkte Kommunikation der Funktionsmodule zum Master möglich, ohne Portsuchen oder andere durch Kommunikationsprotokolle hervorgerufene Verzögerungen.

Die maximale Nutzdatenrate eines Telegramms liegt dadurch bei über 90%, und die theoretische effektive Datenrate durch Ausnutzung der Full-Duplex-Eigenschaft sogar bei über 100 MBit/s (> 90% von zweimal 100 MBit/s). Der EtherCAT-Master ist der einzige Teilnehmer im Segment, der aktiv einen EtherCAT-Frame versenden darf; alle anderen Teilnehmer leiten die Frames nur weiter. Dies vermeidet unvorhersehbare Verzögerungen und garantiert die Echtzeitfähigkeit. Dabei ist zu beachten, dass dies keine "Weiterleitung" im herkömmlichen Sinne bedeutet. Es erfolgt erfindungsgemäß kein Stopp des Signals in einem der Funktionsmodule. Vielmehr ermöglicht die Schaltung, dass die Datenpakete, wie zuvor erwähnt, die einzelnen Module lediglich durchlaufen und die Module zu diesem Zeitpunkt Daten aus dem Frame herunter und in den Frame hochladen können.

Der Master nutzt einen Standard-Ethernet-Medium-Access-Controller (MAC) ohne einen zusätzlichen Kommunikationsprozessor. Damit kann ein Master auf jeder Hardware-Plattform installiert werden, die einen Ethernet-Port zur Verfügung stellt. Das verwendete Echtzeit-Betriebssystem oder die Applikationssoftware sind dabei unerheblich. Die EtherCAT-Slaves nutzen einen EtherCAT Slave Controller (ESC) für die Verarbeitung "on-the-fly". Die Verarbeitung erfolgt also vollkommen in Hardware, wodurch die Performance des Netzwerks berechenbar wird und nicht von der Implementierung der einzelnen Slaves abhängt.

Der Master erstellt die Datenpakete dabei entsprechend der Netzarchitektur. So kann gemäß wenigstens einer bevorzugten Ausführungsform der Erfindung die Konfiguration der Module in dem Bus einmal festgelegt und in dem Master hinterlegt werden. Nach dieser Installation können die Datenpakete entsprechend der tatsächlichen physischen Anordnung in dem Bus-System erstellt und entsprechend verarbeitet werden. Die Module können somit die speziell an das jeweilige Modul gerichteten Befehle herunterladen, und verarbeiten und Daten entsprechend in die durchlaufenden Datenpakete hochladen.

Auf diese Weise wird ermöglicht, dass der erstellte Frame, lediglich beschränkt durch die physische Laufzeit der Datenpakete durch die Leitungen, alle Module quasi simultan erreicht. Ein Frame, also ein derartiges Datenpaket, kann innerhalb von weniger als 100ns, vorzugsweise weniger als 50ns, insbesondere weniger als 20ns, beispielsweise innerhalb von 15ns alle Funktionsmodule in dem Echtzeit-Bus erreichen.

Das EtherCAT Bussystem kann demnach die für die genannten Anwendungen und insbesondere für Anwendungen des erfindungsgemäßen Systems die notwendigen Fähigkeiten aufweisen:
- Die Update-Zeit für die Daten von 1.000 verteilten Ein-/Ausgängen beträgt nur 30 µs - einschließlich Klemmendurchlaufzeit;
- Mit einem einzigen Ethernet-Frame können bis zu 1.486 Byte Prozessdaten ausgetauscht werden - das entspricht fast 12.000 digitalen Ein- und Ausgängen. Für die Übertragung dieser Datenmenge werden dabei nur 300 µs benötigt;
- Performance 256 Digital-I/Os in 12 µs, 1.000 Digital-I/Os in 30 µs, 200 Analog-I/Os (16 Bit) in 50 µs, entspricht 20-kHz-Sampling-Rate, 100 Servoachsen alle 100 µs, 12.000 Digital-I/Os in 350 µs.

In einer bevorzugten Ausführungsform des erfindungsgemäßen Systems wird für die Kommunikationsverbindung zwischen dem Aktionsmodul und dem Erfassungsmodul eine kabelgestützte Datenkommunikation per Lichtwellenleiter oder Kupferkabel eingesetzt. Dies hat den Vorteil, dass die Datenübertragung vergleichsweise zuverlässig, sicher und schnell erfolgt.

In einer vorteilhaften Weiterbildung des erfindungsgemäßen Systems wird für die Kommunikationsverbindung zwischen dem Aktionsmodul und dem Erfassungsmodul bzw. mit dem Mastermodul eine funkgestützte Datenkommunikation nach dem 5G Standard oder dem 6G Standard eingesetzt. Dies hat den Vorteil, dass die Datenübertragung trotz der Nutzung einer Funkverbindung in Echtzeit erfolgen kann.

Gemäß einer vorteilhaften Weiterbildung der Erfindung ist vorgesehen, dass an den echtzeitfähigen Bus, insbesondere EtherCAT-Bus, auch als ECAT bezeichnet, zusätzlich zu dem Aktionsmodul zur Betätigung von Aktoren zur Stimulation von Gewebe und dem Erfassungsmodul zur Ableitung/Messung von Biodaten oder Biosignalen, ein Steuerungsmodul, zur digitalen Input/Output-Steuerung (DIO) von Aktoren externer Geräte, wobei die Aktoren zur Stimulation von Gewebe ausgebildet sind, und ein Mastermodul, zur Verarbeitung der Modul-Signale/daten, angeschlossen sind. Außerdem sind die Module dazu ausgelegt, dass sie auf dem echtzeitfähigen Bus Information im selben Berechnungsschritt in einem vom Mastermodul abhängig von dessen Datenverarbeitung bereitgestellten Takt untereinander austauschen, und dass die vom Erfassungsmodul, vorzugsweise einem A/D-Wandler, gemessenen Daten/Signale zum Mastermodul geschickt und von diesem verarbeitet werden, und das Mastermodul Daten/Befehle an das Aktionsmodul sendet, um über die Aktoren die Stimulation von Gewebe zu aktivieren.

Vorteilhafterweise ist wenigstens ein Funktionsmodul mit einer EtherCAT-Schnittstelle ausgebildet. Auf diese Weise können die Echtzeitbedingungen des Systems, insbesondere die harten oder festen Echtzeitanforderungen an die Vorrichtung, verbessert erreicht werden. Vorteilhafterweise haben eine Mehrzahl, insbesondere alle, Funktionsmodule, die an einer Stimulation, Messung und/oder Auswertung beteiligt sind, eine derartige echtzeitfähige EtherCAT-Schnittstelle. Die Funktionsmodule sind dabei in einer "Master-Slave-Konfiguration" als "Slave" an den Master-Bus, hier den EtherCAT-Bus, angeschlossen.

Mit diesem System wird erreicht, dass innerhalb einer While-Schleife basierend auf dem EtherCAT-Bus, die Module zur Signalmessung/-erfassung, einschließlich zur Erfassung von Biosignalen, wie etwa EEG, EKG, EXG usw., zur digitalen Input/Output-Steuerung peripherer externer Geräte und zur analogen Signalerstellung für die vom Aktionsmodul gesteuerten Aktoren derart ausgelegt werden können, dass sie gleichzeitig bzw. in einem Berechnungsschritt Informationen untereinander auf dem Bus bereitstellen. Dies geschieht typischerweise alle 1 ms aufs Neue, vorgegeben vom Takt des Busses und vom Inhalt der Datenverarbeitung des Mastermoduls.

Vorteilhafte Ausführungsformen der Module des erfindungsgemäßen Systems sehen vor
- dass das Erfassungsmodul(3, Modul A/D) einen A/D-Wandler zur Wandlung von diesem Modul erfasster analoger Biodaten/Biosignale in Digitalsignale umfasst, die im Mastermodul verarbeitet werden; und/oder
- dass das Aktionsmodul einen D/A-Wandler zur Wandlung von vom Mastermodul bereitgestellten digitalen Steuerungssignalen in Analogsignale umfasst, die den Aktoren zur Stimulation von Gewebe zugeführt werden, und/oder
- dass das Steuerungsmodul zur digitalen Input/Output-Steuerung den Signalfluss der vom D/A-Wandler des Aktionsmoduls und der vom Mastermodul bereitgestellten digitalen Steuerungssignale mittels vom Mastermodul erzeugter und vom Steuerungsmodul erzeugter Triggersignale steuert.

Ausgehend von bekannten Verfahren zur körperzustandsabhängigen Stimulation kann eine weitere Aufgabe der Erfindung darin gesehen werden, ein Verfahren anzugeben, mit dem vergleichsweise zuverlässig und sicher eine körperzustandsabhängige Stimulation durchgeführt werden kann.

Ein vorteilhaftes Verfahren zur körperzustandsabhängigen Stimulation auf Grundlage des Systems gemäß einem der Systemansprüche sieht vor,
- dass die Module in einem Closed-Loop (geschlossener Regelkreis) miteinander kommunizieren und die Daten des Aktionsmoduls Auswirkungen auf das Verhalten/die Funktion des Erfassungsmoduls haben und umgekehrt,
- dass die Stimulation aus einer Neuromodulations-Erregung/Hemmung besteht,
- dem das Aktionsmodul magnetische, elektromagnetische, mechanische, pneumatische und/oder hydraulische Aktoren zur direkten Beeinflussung des biologischen Gewebes oder von Organen ansteuert,
- das Aktionsmodul eine mehrkanalige Stimulation biologischen Gewebes auf Basis von Merkmalen aus der Biodatenerfassung von Biosignalen unterschiedlichen Ursprungs durch das Erfassungsmodul in einem Frequenzbereich von 0 bis einigen Kilohertz, insbesondere bis 100, 200 oder 300 kHz durchführt, und
- das Erfassungsmodul dazu ausgelegt ist, EEG-, EKG-, EXG-, EMG-, EOG-, ERG-, PPT-, Atmungs-, MKG-, MEG-, BD-, SpO2-Signale zu erfassen.

Die Offenbarung betrifft des Weiteren auch eine Vorrichtung aufweisend ein Steuerungsmodul bzw. Mastermodul, einen Echtzeit-Master-Bus, eine Mehrzahl Modulsteckplätze, vorzugsweise wenigstens zwei, jedoch mindestens einen Modulsteckplatz, sowie wenigstens ein Funktionsmodul, wobei der wenigstens eine der Modulsteckplätze mit einer echtzeitfähigen Datenverbindung mit dem Mastermodul 4 verbunden ist und das wenigstens eine Funktionsmodul mit einer echtzeitfähigen Schnittstelle ausgebildet und mit dem echtzeitfähigen Master-Bus verbunden ist. Dabei kann die Echtzeitfähigkeit derart vorgesehen sein, dass harte oder feste Echtzeitbedingungen erfüllt werden. Des Weiteren ist das wenigstens eine Funktionsmodul mittels Hard- oder Software dazu ausgebildet, die zu verarbeitenden Daten an nachgeschaltete interne und/oder externe Komponenten, beispielsweise Datenerfassungsvorrichtungen, Messeinheiten, Ausgabegeräte etc. zu senden, vorzugsweise ebenfalls unter denselben Echtzeitbedingungen.

Durch die Rückkopplung und Auswertung bestimmter Merkmale aus Biosignalen lassen sich Modulationen signal- oder statusabhängig verändern. Die spezifischen Parameter hängen von dem konkreten Anwendungsfall ab. Beispiele hierfür sind: die Analyse der individuellen Alphafrequenz im EEG und die Auslösung eines Triggersignals für ein TMS-Gerät bei einer Phase von 90° im EEG; oder die Analyse eines Herzschlages im EKG und die Auslösung eines Stromimpulses im systolischen Intervall. Dabei kann im EEG beispielsweise die Amplitude, die Frequenz und die Phase analysiert werden. Im EKG kann beispielsweise die Amplitude, der Zeitpunkt der R-Zacke und der zeitliche Abstand zwischen R-Zacken analysiert werden. Nachfolgend werden unter Bezugnahme auf die beigefügten Figuren vorteilhafte Weiterbildungen und weitere Ausführungsbeispiele näher beschrieben und erläutert.

Es zeigen:
Fig. 1 ein integriertes Stimulations- und Messsystem (MIS);
Fig. 2 eine erste Modulübersicht verschiedener Module des MIS;
Fig. 3 eine zweite Modulübersicht verschiedener Module des MIS;
Fig. 4 ein Beispiel einer Closed-Loop Sequenz für eine Ausführungsform des integrierten Stimulations- und Messsystems;
Fig. 5 eine Detektion eines Amplitudenmaximums eines synthetischen Sinussignals;
Fig. 6 eine Detektion des Amplitudenmaximums eines EEG-Signals; und
Fig. 7 ein Beispiel für eine erfindungsgemäße Vorrichtung mit einem integrierten Stimulations- und Messsystem.

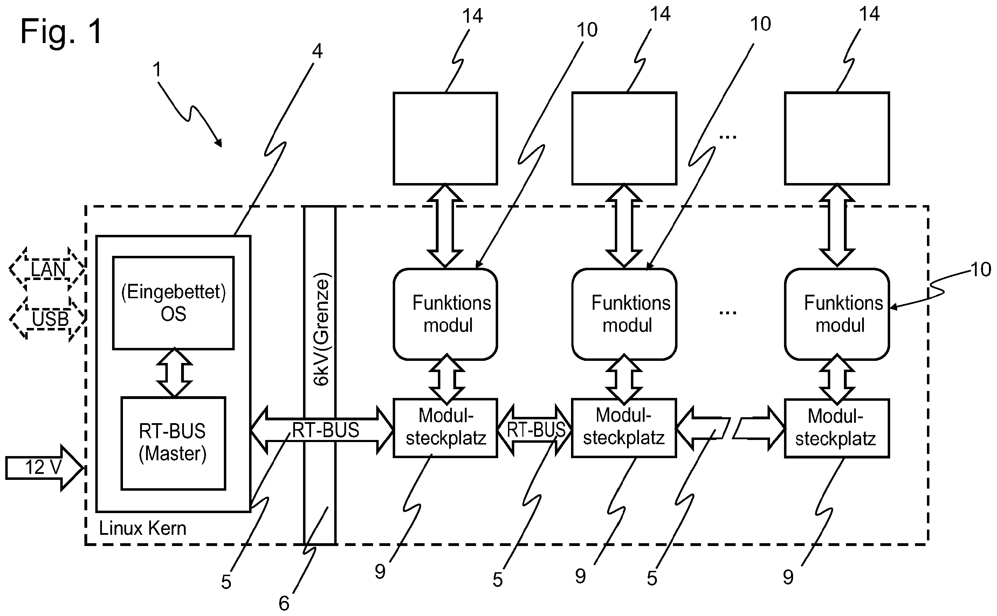

Fig. 1 zeigt eine Schemadarstellung eines erfindungsgemäßen integrierten Stimulations- und Messsystems (MIS) 1. Das MIS 1 kann als eine Vorrichtung ausgebildet sein, die eine Gehäuse aufweist, in Fig. 1 angedeutet durch die gestrichelte umlaufende Rahmenlinie. Das MIS 1 weist ein Signalverarbeitungsmodul 4, auf. Das Signalverarbeitungsmodul 4 hat einen Echtzeit-Bus Master und eine Recheneinheit. Die Recheneinheit kann ein Computerchip sein, mit einem darauf installierten Betriebssystem. Vorteilhafterweise weist das Signalverarbeitungsmodul 4 einen Linuxkern mit eingebettetem Betriebssystem (Embedded OS) auf. Das Signalverarbeitungsmodul 4 auch als Mastermodul bezeichnet, kann beispielsweise ein so genanntes Embedded Board aufweisen, mit einer Taktung von 800MHz - 1GHz. Es versteht sich, dass auch andere Taktungen denkbar sind, soweit diese harte oder feste Echtzeitanforderungen erfüllen, die für die entsprechend vorgesehenen Anforderungen gelten.

Der Echtzeit-Bus Master, in Fig. 1 auch als RT-BUS Master (real-time bus master) bezeichnet, ist über einen Echtzeit-Bus 5 mit einer Mehrzahl von Modulsteckplätzen 9 verbunden. Jeder Modulsteckplatz 9 ist zur Aufnahme eines Funktionsmoduls 10 ausgebildet und vorgesehen. Unter einem Funktionsmodul 10 soll dabei jedes Modul verstanden werden, das ausgebildet ist, die Funktion des MIS zu ermöglichen oder dessen Funktionalität zu erweitern.

In Fig. 1 sind beispielhaft drei Modulsteckplätze dargestellt, es können jedoch auch mehr oder weniger Module in dem MIS vorgesehen sein. So können, für einen erfindungsgemäßen Gebrauch des MIS, beispielsweise mindestens zwei Funktionsmodule 10 vorgesehen sein. Die Funktionsmodule 10 sind wiederum verbunden mit integrierten oder externen Komponenten 14. Diese Komponenten 14 können beispielsweise Aktoren, Erfassungsgeräte, Anzeigevorrichtungen, Elektroden, etc. sein. Beispielsweise können die Komponenten 14 folgende Funktionen ermöglichen oder realisieren: EEG Messungen, Setzen von Triggersignalen und/oder Auslesen von Triggersignalen, Stromversorgung des MIS und/oder einer oder mehrerer der Komponenten 14, Datenerfassung, Datenverarbeitung und/oder Datentransfer, Anzeige von Daten, Funktionsmenus oder anderer Informationen, Wartungs- und/oder Steuerungsfunktionen für das MIS und/oder eine der angeschlossenen Komponenten 14 und/oder Bedienungsfunktionalitäten, insbesondere eine berührungssensitive Steuerung einer integrierten Anzeigevorrichtung.

Die in Fig. 1 gezeigten Doppelpfeile mit durchgezogener Linie stellen Kommunikationsverbindungen dar, die vorzugsweise Echtzeitbedingungen erfüllen. So ist die Kommunikationsverbindung zwischen einem Modulsteckplatz 9 und einem Funktionsmodul 10 zwingend an harte oder fest Echtzeitanforderungen gebunden, um den erfindungsgemäßen Echtzeit-Betrieb des MIS zu ermöglichen. Die in der Fig. 1 gezeigten Doppelpfeile mit gestrichelten Linien stellen weitere Datenverbindungsschnittstellen dar, die üblicherweise keine Echtzeitanforderungen erfüllen, hier eine USB Schnittstelle und eine LAN-Schnittstelle. Zudem ist in Fig. 1 eine Stromquelle von 12V angedeutet.

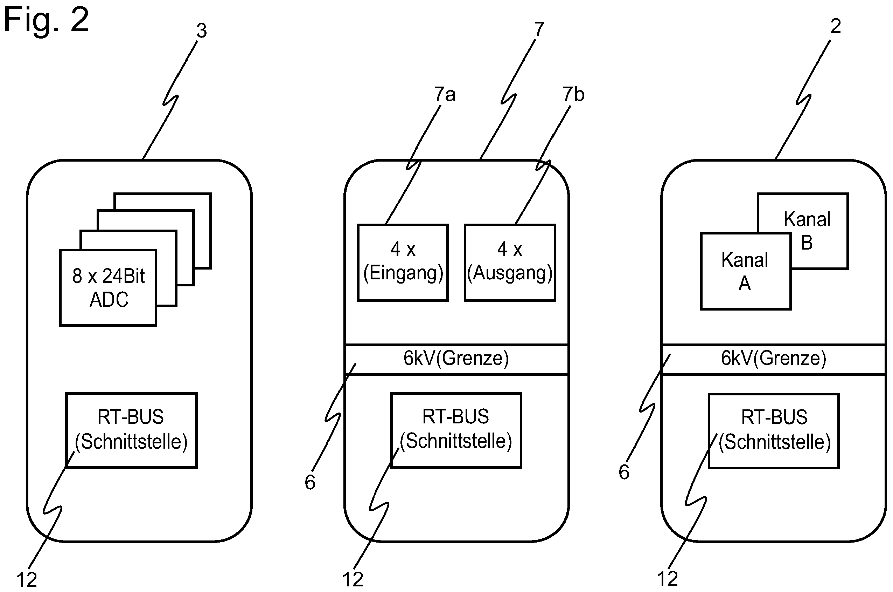

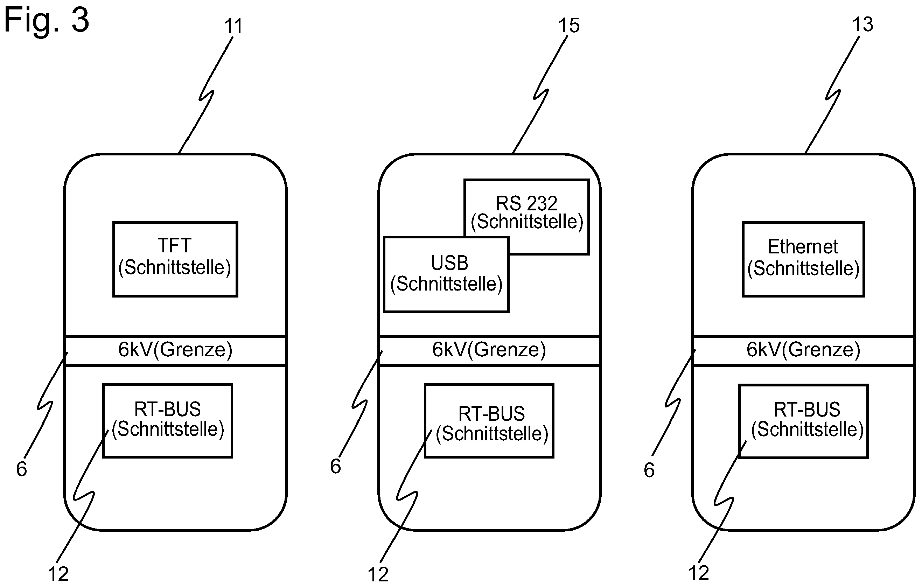

Wie in der Ausführungsform nach Fig. 1 gezeigt, ist das integrierte Stimulations- und Messsystem (MIS) 1 in Form von Funktionsmodulen 10 realisiert, die sich sowohl auf der Erfassungs- (Erfassungsmodul 3) aber auch Ausgabenseite (Aktionsmodul 2) beliebig erweitern lassen, ohne die Kapazitäten zur zeitgenauen und akkuraten Datenverarbeitung untereinander zu beeinträchtigen (vgl. Fig. 2 und 3). Die Module kommunizieren über die gemeinsame Kommunikationsverbindung 5, einen Echtzeitbus. Die Funktionsmodule 10 können der Stromstimulation, Ansteuerung von elektrischen, mechanischen bzw. pneumatischen Aktoren, Auslösung von Ereignissen, Erfassung von Daten, Ausgabe und Anzeige von Daten und anderem dienen. Ein Funktionsmodul kann beispielsweise auch zur Grafikverarbeitung, als Stromquellenmodul oder als 3D-Beschleunigermodul ausgebildet sein.

Somit ist das MIS 1 eine generische Plattform, die auch Untersuchungen in anderen Gebieten der Medizintechnik durch eine Erweiterung des Parameterbereichs für die Erfassung von Biosignalen und die Entwicklung anderer Formen der Ausgabe erlauben kann. Dies kann beispielsweise durch Integration weiterer Funktionsmodule, die zur Erfassung vordefinierter Parameter ausgebildet sind, erfolgen. Zudem ist es denkbar, dass vorhandene Funktionsmodule durch Anpassung der Ansteuerung und/oder deren Programmierung in ihrem Funktionsumfang verändert oder erweitert werden.

In vorteilhaften Weiterbildungen der Erfindung, kann das erfindungsgemäße MIS 1 für eine oder mehrere der folgenden Anwendungen ausgebildet sein:
- Einprägung des Hirnrhythmus von außen durch die transkranielle Wechselstromstimulation (tACS) und Auslösung einer phasenbezogenen TMS zur gezielten Hemmung oder Erregung kortikospinaler Bahnen in Echtzeit;
- Messung der individuellen EEG-Alpha-Peak-Frequenz (iAPF) und repetitive Ansteuerung des TMS-Gerätes mit dieser Frequenz für wissenschaftliche Studien bzgl. der Behandlung von Menschen mit Depressionen in Echtzeit;
- Erfassung des EEG und Auslösung eines phasenbezogenen Ereignisses, z. B. TMS-Puls, für wissenschaftliche Studien bzgl. der Behandlung von Menschen mit Depressionen in Echtzeit;
- Erfassung des EEG und phasenbezogene elektrische periphere Stimulation (FES) zur Rehabilitation;
- Erfassung des Blutdrucks (BP / BD) und phasenbezogene elektrische periphere Stimulation für die Schmerzbehandlung;
- Erfassung des Atems oder von Atemsignalen und daran angepasste Stimulation des Zwerchfells.

Basierend auf dem MIS kann eine feedback-gekoppelte Modulation (Stimulation) der Hirnfunktion auf der Basis der individuellen, in Echtzeit heran gezogenen Patientenphysiologie ermöglicht werden. Untersuchungen zeigen, dass aufgrund der hier vorgeschlagenen Realisierung nicht nur eine schnellere Anwendung, erfindungsgemäß in einer Closed-Loop Schaltung, also einem geschlossenen Regelkreis mit Raten von < 1 bis 3 ms für Erfassung und Verarbeitung des EEG und Auslösens eines Ereignisses, ermöglicht werden kann. Es kann auch eine höhere zeitliche Genauigkeit bei der Erfassung und wiederholten Stimulation ermöglicht werden, ohne dass eine Vorhersage in die Zukunft, auch als Prädiktion bezeichnet, notwendig ist, wie bei gegenwärtigen Systeme üblich. Die Phasenabweichung von den gewünschten Triggerzeitpunkten basierend auf den erfassten Messdaten, die mit einem erfindungsgemäßen MIS ermöglicht werden kann, kann frequenzabhängig bei einer EEG Messung bei einer Frequenz von 4Hz beispielsweise bei +/- 5°, bei einer Frequenz von 40Hz bei +/- 12° liegen. Bei einer EKG Detektion des systolischen Intervalls kann eine Abweichung beispielsweise bei +/-3ms liegen.

Wesentliche Vorteile und vorteilhafte Weiterbildungen dieser Anordnung, insbesondere gegenüber herkömmlichen Lösungen, können die im Folgenden genannten sein:
- Die komplette Signalverarbeitungskette (Datenerfassung - Weiterleitung - Verarbeitung - Weiterleitung - Aktion) ist in den Echtzeitbus eingebettet und kann durch diesen synchronisiert werden;
- die vier Stufen können durch den Echtzeitbus in allen Modulen gleichzeitig (nur gebremst durch die Signallaufzeit auf dem Bus) mit neuen Daten beschickt werden;
- durch das Paketintervall (Takt des Busses) ergibt sich eine maximale Verzögerung zwischen Datenerfassung und Aktion von einem Paketintervall auf dem Bus;
- die Erfassung (mit einem Erfassungsmodul 3) kann an alle vorstellbaren Signale biologischen Ursprungs anpassbar sein, da die Schnittstelle zum Bus für alle Module gleich sein kann;
- das Ansteuern von Aktoren zur Stimulation biologischen Gewebes (mit einem Aktionsmodul 2) bedarf keiner Prädiktion, da auch diese über eine Schnittstelle an den Bus angekoppelt sein können;
- modularer Aufbau und beliebige Kombination von Erfassungs- 3 und Aktionsmodulen 2. Einzig die Verarbeitungssoftware muss an die jeweilige Aufgabe angepasst werden;
- die Verarbeitung der Daten und die Steuerung des Echtzeit-Busses kann in einem Embedded Linux OS (z. B. Toradex-SOM) direkt im Gerät ausgeführt werden.

In den Figuren 2 und 3 sind Beispiele für (Funktions-)Module gezeigt, die an den Echtzeit-Bus (RT-BUS) angeschlossen werden können.

Fig. 2 zeigt als ein erstes Beispiel für ein Funktionsmodul 10 ein Erfassungsmodul 3, hier als EEG-Modul zur Datenerfassung (beispielsweise ADS 1299) und Anbindung an den Bus ausgeführt. Dieses Erfassungsmodul 3, sowie auch die übrigen Funktionsmodule 10, weist einen Schnittstellenabschnitt oder auch Funktionsabschnitt mit Schnittstellen für externe Geräte 14, sowie einen Verbindungs- und Steuerabschnitt (RT-BUS Interface) 12 auf. Der Verbindungs- und Steuerabschnitt 12 weist in wenigstens einer bevorzugten Ausführungsform einen Mikrocontroller (µC) (beispielsweise Infineon XMC48xx) auf.

Der Schnittstellenabschnitt weist in der gezeigten Ausführungsform vier Schnittstellen mit jeweils 8 Kanälen auf, ermöglicht also bis zu 32 Kanäle beispielsweise für eine EEG-Messung. Jeder Kanal arbeitet mit 24Bit Auflösung. In alternativen Ausführungsformen können die Kanäle auch mit anderer Auflösung arbeiten bzw. betrieben werden. Das EEG Modul 3 ist dabei ein Analog/Digital Konverter (ADC) und wird im Folgenden auch als A/D Modul bezeichnet. Das Erfassungsmodul 3 ist für eine Samplerate von 1000 Sample/sek vorgesehen, was pro Sample bzw. Datenpaket eine Zeit von 1ms erlaubt. Die Schnittstelle des Funktionsabschnitts zu einer externen Komponente kann dabei kabelgebunden, aber in vorteilhafter Weiterbildung auch drahtlos, basierend auf einem ausreichend schnellen Kommunikationsstandard wie 5G oder 6G oder auch optisch, sein. Dies gilt analog auch für die anderen Funktionsmodule.

Das RT-BUS Interface ist dazu vorgesehen und ausgebildet, in einen Modulsteckplatz des MIS eingebracht zu werden. Auf diese Weise wird die Verbindung mit dem Echtzeit-Bus des MIS hergestellt und das Funktionsmodul in das MIS integriert.

Das RT-BUS Interface 12 des Erfassungsmoduls 3 weist dabei eine speziell für das Erfassungsmodul 3 erstellte Konfiguration auf. Der Mikrocontroller dient dabei der Steuerung des Funktionsmoduls 10 und der Datenanbindung und Datenverarbeitung für den Echtzeitbetrieb.

Einen analogen Aufbau, also Funktionsabschnitt und Verbindungs- und Steuerabschnitt weisen auch die übrigen (Funktions-)Module auf, die in das MIS integriert sind.

Fig. 2 zeigt als ein weiteres Beispiel für ein Funktionsmodul 10, ein Steuerungsmodul 7, auch als IO-Modul oder DIO-Modul bezeichnet. Das Steuerungsmodul 7 dient zur Anbindung an den Bus über einen Mikrocontroller (z.B., Infineon XMC48xx). Das hier gezeigte Steuerungsmodul 7 kann insbesondere zum Auslösen oder Empfangen eines oder mehrerer Triggersignale ausgebildet sein und/oder zum Setzen eines entsprechenden Pegels für das Triggersignal. Das Steuerungsmodul 7 weist in der gezeigten Ausführung 4 Dateneingänge 7a und 4 Datenausgänge 7b auf. Dabei kann bevorzugt eine Transistor-Transistor Logik (TTL) vorgesehen sein. Die Ein- bzw. Ausgänge 7a, 7b sind wiederum durch eine galvanische Trennung 6 mittels eines digitalen Isolators (z. B. ISOW78xx Infineon) von dem Bus getrennt. Die galvanische Trennung kann dabei hier und auch bei den anderen Modulen und galvanischen Trennungen beispielsweise einer 6kV-Barriere entsprechen.

Die hier und in den Funktionsmodulen vorteilhafterweise vorgesehenen galvanischen Trennungen ermöglichen es, einen Patienten von der Messeinrichtung elektrisch zu isolieren, bzw. die einzelnen Komponenten der Messeinrichtung voneinander zu isolieren. Dies kann Störsignale und fehlerhafte Messungen reduzieren.

Fig. 2 zeigt des Weiteren ein Aktionsmodul 2, hier als StromModul oder Current-Modul bezeichnet. Das Current-Modul dient durch Ausgabe von Strömen als Aktor zur Stimulation. Das Current-Modul ist hier mit zwei Kanälen (Channel A und Channel B) dargestellt. Im vorgeschlagenen Aufbau werden Stromquellen verwendet, die getrennt und somit auch in der Energiezufuhr unabhängig sind. So kann ein geringeres Interferieren der Stromquellen erreicht werden. Die Stromquellen können dabei selbstkalibrierend, rauschärmer und in Echtzeit, beispielsweise innerhalb 1 ms mit neuen Parametern ansteuerbar sein. Eine Datenpufferung kann daher entfallen. Das RT-BUS Interface ist wiederum galvanisch von dem Schnittstellenabschnitt getrennt. Abhängig von der spezifisch gewünschten Ausführungsform hinsichtlich einer vorbestimmten Anwendung, kann als Echtzeitintervall auch eine Dauer von mehr oder weniger als 1ms gewählt werden.

Fig. 3 zeigt ein Funktionsmodul, das als Anzeigevorrichtung, speziell als TFT-Modul 11 ausgebildet ist. Das TFT-Modul 11 dient zur Anbindung einer Anzeigevorrichtung an den Bus. Wie zuvor beschrieben erfolgt die Anbindung über einen Mikrocontroller (z.B. Infineon XMC48xx) mit galvanischer Trennung der Anzeigevorrichtung über einen digitalen Isolator der Serie ADUM1xx (Analog Devices). In der gezeigten Ausführung weist das TFT-Modul 11 eine Ansteuerung für einen TFT Bildschirm über FT813 auf. Die Auflösung für die Anzeigevorrichtung beträgt dabei hier 800 x 400 Pixel und diese weist eine Berührungserkennung auf.

In wenigstens einer Fortbildung der Erfindung, ist die Anzeigevorrichtung fest in das MIS integriert. Es versteht sich, dass auch andere Anzeigevorrichtung eingebunden werden können, die fest integriert oder als externe Geräte vorgesehen sind. Dabei können die Anzeigevorrichtungen verschiedene Eigenschaften haben, beispielsweise mit Berührungserkennung oder ohne ausgebildet sein, mit verschiedenen Auflösungen, monochromatisch oder farbig, etc. Zudem kann auch eine Mehrzahl an Anzeigevorrichtungen vorgesehen sein.

Fig. 3 zeigt weiter ein Funktionsmodul, das als COM-Modul (Kommunikationsmodul) 15 ausgebildet ist. Das COM-Modul 15 dient der Anbindung anderer, auch nicht echtzeitfähiger, Schnittstellen, wie beispielsweiser einer oder mehrerer USB- oder RS232-Schnittstellen. Es sind auch weitere Schnittstellen denkbar, beispielsweise eine oder mehrere CAN-Bus Schnittstellen, und insbesondere auch kabellose Verbindungsschnittstellen zur Verbindung mit externen Geräten.

Ein weiteres Beispiel für ein mit dem MIS 1 verbindbares Modul ist, wie in Fig. 3 gezeigt, ein LAN-Modul (Netzwerkmodul) 13. Das LAN-Modul 13 dient zur Anbindung an ein herkömmliches lokales Netzwerk (Ethernet). Dafür weist das LAN-Modul 13 eine entsprechende LAN-Schnittstelle und eine Buchse für ein Datenkabel auf. Die Schnittstelle ist vorliegend eine Schnittstelle für ein 100Mbit LAN. Es versteht sich, dass die Schnittstelle auch für andere Übertragungsraten ausgebildet sein kann.

Ein weiteres Funktionsmodul 10 kann beispielsweise ein Strommodul zur Verbindung des MIS mit einer Stromquelle sein. Es ist zudem denkbar, dass eines der Module als Erfassungsmodul mit einer Schnittstelle zu einem Beschleunigungssensor ausgebildet ist. Ein Beschleunigungssensor kann beispielsweise zur Erfassung einer oder mehrerer Frequenzen eines Tremors dienen, wie er bei Parkinson-Erkrankungen in der Regel auftritt. Auch weitere Schnittstellen und Funktionsmodule sind denkbar, um diese in Echtzeitumgebung, die erfindungsgemäß ermöglicht wird, einzubinden.

Die Datenverarbeitung in dem MIS 1 erfolgt vollständig digital. Eine gleichzeitige körperzustandsabhängige Stimulation und Erfassung von Biosignalen unterschiedlichen Ursprungs ist mit verschiedenen Verstärkungsfaktoren und Abtastraten möglich. Die modulare Konzeption der Funktionsmodule über die gemeinsame digitale Schnittstelle über den Echtzeitbus ermöglichen eine beliebige Kaskadierbarkeit.

Die Daten werden nicht - wie in konventionellen Systemen üblich - per Zeitmultiplex erfasst, sondern können auf Grund der modularen Struktur auch simultan erfasst, aber auch völlig unabhängig voneinander abgetastet werden. So kann erfindungsgemäß ermöglicht sein, einen Stimulationsimpuls innerhalb geringster Toleranzgrenzen bei einer vorbestimmten Phase eines Messsignals auszulösen, während das Signal erfasst wird und beispielsweise ein Schmerzreiz bei einem Patienten ausgelöst wird.

Zur Ausführung einer Messung mit Gewebestimulation unter Echtzeitbedingungen sind beispielsweise wenigstens das Mastermodul 4 und zwei weitere Funktionsmodule 10 notwendig, insbesondere das Erfassungsmodul 3 zum Erfassen der Messwerte, und das Aktionsmodul 3zum Stimulieren eines Gewebes.

Zur Ausführung einer Messung mit Triggersignal unter Echtzeitbedingungen sind beispielsweise wenigstens das Mastermodul 4 und zwei weitere Funktionsmodule 10 notwendig, insbesondere das Erfassungsmodul 3 zum Erfassen der Messwerte, und das Steuerungsmodul 7 zum Auslösen eines Triggersignals.

Die digitalen Schnittstellen zwischen den Funktionsmodulen ermöglichen eine sehr effiziente galvanische Trennung 6 der Messanordnung von der Ausgabe- und Auswertetechnik, so dass aufwendige analoge Isolationsverstärker zur Gewährleistung der technischen Sicherheit bei medizinischem Einsatz entfallen können, ohne dass dadurch die Sicherheit gegenüber dem Messsubjekt (Patient) eingeschränkt wird. Die Einhaltung der Norm EN 60601-1 über die Allgemeine Festlegungen für die Sicherheit einschließlich der wesentlichen Leistungsmerkmale ist somit gewährleistet.

Im Vergleich mit der herkömmlichen Technik zeichnet sich die vorgeschlagene Lösung durch eine geringe Baugröße und einen niedrigen Energiebedarf aus. Dies liegt bereits darin begründet, dass nur eine zentrale Rechnungseinheit, nämlich das Mastermodul 4, notwendig ist, anstatt mehrerer separater miteinander vernetzter Computer. Alle für die Messungen, und Stimulationen notwendigen Module können erfindungsgemäß in einer Vorrichtung innerhalb eines hier nicht gezeigten Gehäuses vereint sein.

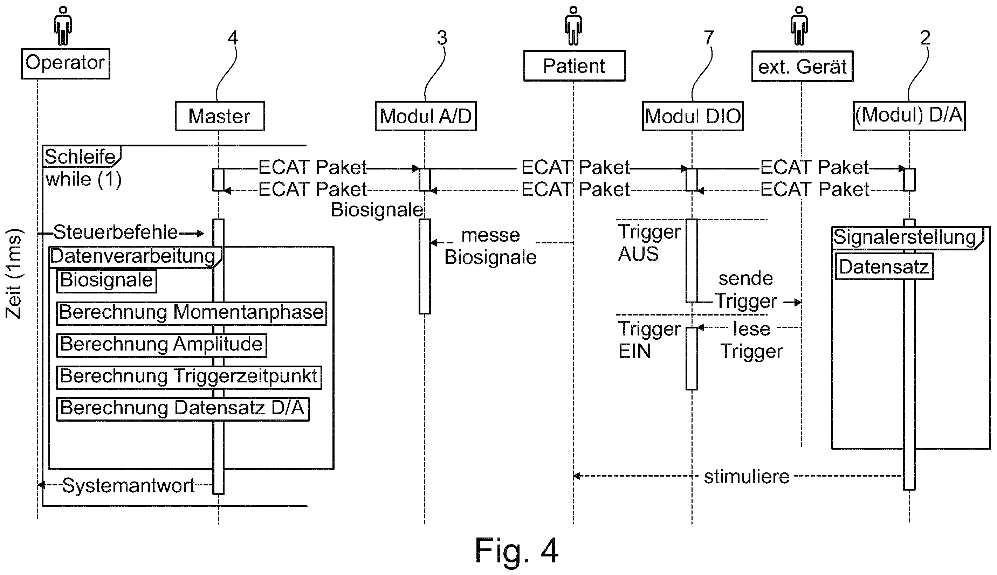

Fig. 4 zeigt ein Beispiel einer Closed-Loop Sequenz für eine Ausführungsform des integrierten Stimulations- und Messsystems, das einen Echtzeitbus umfasst. Der Echtzeitbus ist bevorzugt ein EtherCAT-Bus, in der Figur auch als ECAT bezeichnet.

An den EtherCAT-Bus ECAT sind zusätzlich zu dem Aktionsmodul 2 des Echtzeitbusses von Fig. 1 zur Betätigung von Aktoren zur Stimulation von Gewebe, in Fig. 4 als modul D/A bezeichnet, und dem Erfassungsmodul 3 des Echtzeitbusses von Fig. 1, in Fig. 4 als modul A/D zur Ableitung/Messung von Biodaten oder Biosignalen bezeichnet, ein Steuerungsmodul 7, in Fig. 4 als modul DIO zur digitalen Input/Output-Steuerung von Aktoren, zur Stimulation von Gewebe, externer Geräte bezeichnet, und ein Mastermodul 4, in Fig.4 auch als master bezeichnet, zur Verarbeitung der Modul-Signale/Daten angeschlossen.

Die Module des EthernetCAT-Busses ECAT sind dazu ausgelegt, dass sie auf dem echtzeitfähigen Bus (ECAT) Information im selben Berechnungsschritt in einem vom Mastermodul 4 (master) abhängig von dessen Datenverarbeitung bereitgestellten Takt untereinander austauschen.

Die vom Erfassungsmodul 3 (modul A/D) gemessenen Daten/Signale werden zum Mastermodul 4 (master) geschickt und von diesem verarbeitet, und das Mastermodul 4 sendet Daten/Befehle an das Aktionsmodul 2 (modul D/A), um über die damit verbundenen Aktoren die Stimulation von Gewebe zu aktivieren.

Das Erfassungsmodul 3 (modul A/D) umfasst einen A/D-Wandler zur Wandlung von in diesem Modul 3 erfassten analogen Biodaten/Biosignale in Digitalsignale, die im Mastermodul 4 (master) verarbeitet werden.

Das Aktionsmodul modul D/A umfasst einen D/A-Wandler zur Wandlung von vom Mastermodul master bereitgestellten digitalen Steuerungssignalen in Analogsignale, die den Aktoren zur Stimulation von Gewebe zugeführt werden.

Das Steuerungsmodul 7 (modul DIO) führt eine digitale Input/Output-Steuerung des Signalflusses der vom D/A-Wandler des Aktionsmoduls 2 (modul D/A) und der vom Mastermodul 4 (master) bereitgestellten digitalen Steuerungssignale mittels vom Mastermodul 4 (master) erzeugter und vom Steuerungsmodul 7 (modul DIO) erzeugter Triggersignale durch.

Mit dem in Fig. 4 gezeigten System wird erreicht, dass innerhalb einer While-Schleife basierend auf dem EtherCAT-Bus, die Module zur Signalmessung/-erfassung, einschließlich zur Erfassung von Biosignalen, wie etwa EEG, EKG, EXG usw., zur digitalen Input/Output-Steuerung peripherer externer Geräte und zur analogen Signalerstellung für die vom Aktionsmodul gesteuerten Aktoren derart ausgelegt werden können, dass sie gleichzeitig bzw. in einem Berechnungsschritt Informationen untereinander bzw. füreinander auf dem Bus bereitstellen. Dies geschieht typischerweise alle 1 ms aufs Neue, vorgegeben vom Takt des Busses und vom Inhalt der Datenverarbeitung des Mastermoduls.

Fig. 4 zeigt dabei eine exemplarische Datenschleife der Messvorrichtung. Dabei ist in einer vertikalen Richtung der Zeitverlauf von oben nach unten dargestellt. Die hier dargestellte Zeit für eine derartige Datenschleife ist 1ms, was einer Frequenz von 1000Hz entspricht.

Es sind in anderen Ausführungsformen der Erfindung natürlich auch andere Frequenzen denkbar, ohne sich von dem erfindungsgemäßen Gedanken zu entfernen, dass hier Echtzeitbedingungen eingehalten werden sollen. Horizontal auf gleicher Höhe angeordnete Ereignisse in Fig. 4 finden dabei simultan oder zumindest annähernd gleichzeitig statt.

Wie zuvor beschrieben, wird von dem Mastermodul 4 ein Datenpaket erzeugt, das über den echtzeitfähigen Bus, insbesondere den EtherCAT Bus, zu den Funktionsmodulen 10, hier dem Erfassungsmodul 3, dem Steuerungsmodul 7 und dem Aktionsmodul 2, gesendet wird. Der Echtzeit Bus ist dabei derart ausgelegt, dass die Datenpakete bildlich gesprochen durch die Funktionsmodule laufen. Während dieses Durchlaufs werden Daten von den Funktionsmodulen aus dem Datenpaket gelesen und hineingeschrieben. Die Datenpakete werden von den Funktionsmodulen also nicht zwischengespeichert und erst anschließend weitergesendet. Auf dieses Weise kann ein quasisimultaner Empfang der Datenpakete bei allen Funktionsmodulen ermöglicht werden.

Jedes der Module ist ausgelegt, auf dem echtzeitfähigen Bus (ECAT) Information im selben Berechnungsschritt in einem vom Mastermodul 4, abhängig von dessen Datenverarbeitung, bereitgestellten Takt Informationen untereinander mittels einer echtzeitfähigen Kommunikationsverbindung 5 auszutauschen. Das Mastermodul 4 ist in dieser Anordnung das einzige Modul, das einen Frame, also ein Datenpaket, erzeugen kann, während die dem Mastermodul 4 nachgeschalteten Funktionsmodule 10 diesen Frame nur auslesen und mit eigenen Daten ergänzen können.

So wird in der in Fig. 4 gezeigten Closed-Loop Anordnung ein Datenpaket von dem Mastermodul erzeugt und an das erste Funktionsmodul, hier das Erfassungsmodul 3, gesendet. In dem Datenpaket kann beispielsweise ein Befehl für das Erfassungsmodul 3 enthalten sein, eine Erfassung von Daten zu beginnen. Das Erfassungsmodul beginnt dann eine Datenerfassung, vorliegend von Biosignalen eines Patienten.

Das Datenframe läuft dabei bereits von dem Erfassungsmodul 3 weiter an ein nachfolgendes Funktionsmodul 10, in Fig. 4 das Steuerungsmodul 7, auch als modul DIO bezeichnet. Das Steuerungsmodul 7 steuert beispielsweise den digitalen Input/Output zu Aktoren der Vorrichtung. Dabei erzeugt das Steuerungsmodul 7 beispielsweise einen Trigger, sendet diesen an ein externes Gerät und/oder liest einen Trigger ein, während das Datenpaket bereits weiter zu dem hier nachgeschalteten Aktionsmodul 2 gelaufen ist.

Das Aktionsmodul 2 empfängt das Datenpaket, erkennt in dem Frame die für Aktionsmodul 2 relevanten digitalen Steuerungsbefehle des Mastermoduls 4 und wandelt diese in ein analoges Signal für an das Aktionsmodul 2 angeschlossene Aktoren um, erstellt ein Signal und beginnt mit einer Stimulation eines Patienten, bzw. sendet einen Datensatz mit Stimulationsdaten an ein externes Gerät, das zur Stimulation mit einem Patienten verbunden sein kann. Noch bevor die Stimulation erfolgt, ist, wie aus Fig. 4 ersichtlich, das Datenpaket zurück zu dem Mastermodul 4 gelaufen.

Das Mastermodul 4 sendet die Frames bzw. Datenpakete mit einer vorgegebenen Rate, beispielsweise ein Paket pro ms, die kontinuierlich in Echtzeit durch die Closed-Loop gesendet werden.

So können die Daten, die das Erfassungsmodul 3 erfasst, einem Datenpaket angehängt und zur Verarbeitung an das Mastermodul weitergeleitet werden, so dass das Mastermodul darauf basierend neue Steuerbefehle für das Steuerungsmodul und das Aktionsmodul in einem der folgenden Frames ausgeben kann, wenn notwendig.

Das Erfassungsmodul 3 weist dabei in der gezeigten Ausführung einen A/D Wandler auf, um die analog erfassten Daten, hier die Biosignale eines Patienten, in ein digitales Signal zur weiteren Verarbeitung umzuwandeln.

Die Datenverarbeitung durch das Mastermodul 4 kann dabei eine Berechnung einer Momentanphase, einer Amplitude, eines Triggerzeitpunktes oder eines Digital/Analog Datensatzes beinhalten. So kann das Mastermodul 4 basierend auf den empfangenen Daten Triggerzeitpunkte definieren, verändern oder anpassen. Es ist auch denkbar, dass auf diese Weise beispielsweise Triggerkaskaden, adaptiert an die Biodaten, erstellt werden. Zudem können mit dem erfindungsgemäßen Echtzeit-System, bzw. dem erfindungsgemäßen Gerät, Kombinationen von Triggersignalen, Erfassungsdaten und Stimulationsdaten auf bislang nicht erreichbaren Zeitskalen kombiniert werden. Dies kann eine verbesserte Analyse biologischer, biophysikalischer und/oder biochemischer Phänomene ermöglichen.

Das Mastermodul 4 kann mit einem Operator verbunden sein und von diesem Steuerbefehle erhalten. Der Operator kann beispielsweise eine die Messung durchführende Person oder eine, beispielsweise digitale, Steuervorrichtung sein. So können vorteilhafterweise auch halb- oder vollautomatisierte Messungen durchgeführt werden. Das Mastermodul 4 gibt, basierend auf der Datenverarbeitung, eine Systemantwort an den Operator aus.

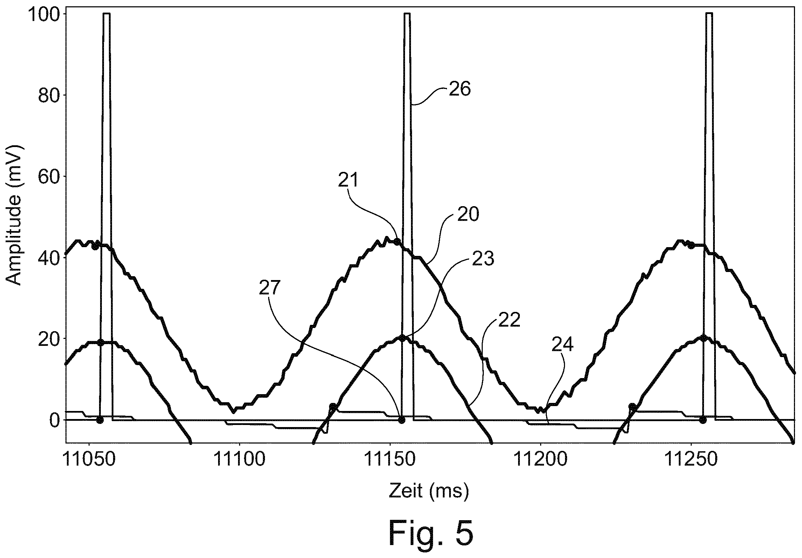

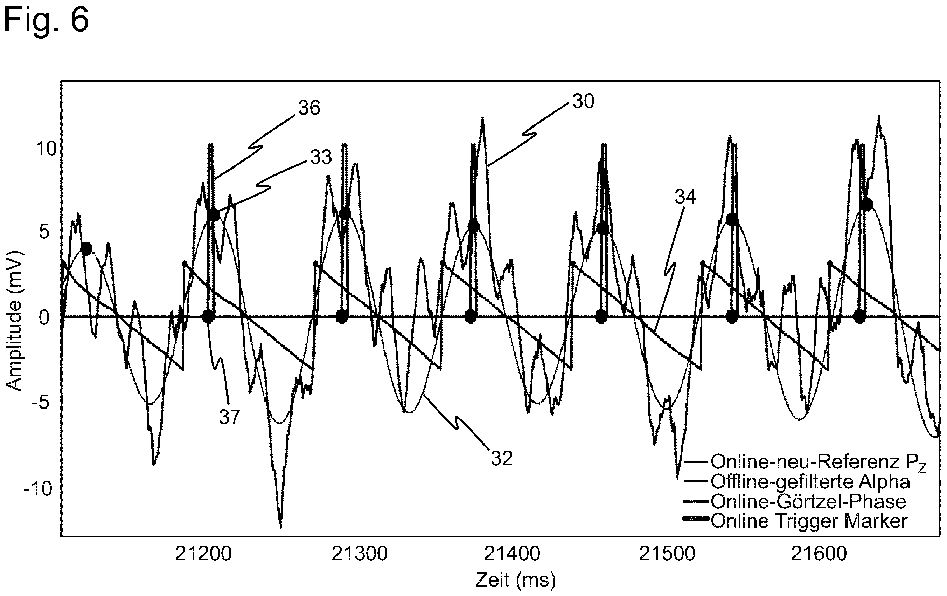

In den Abbildungen der Figuren 5 und 6 sind Messungen eines synthetischen Signals (Figur 5) und eines EEG-Signals (Figur 6) bei der Detektion der Maximalamplitude der Sinusschwingung (Phase: 90°) gezeigt:
Figur 5 zeigt die Detektion des Amplitudenmaximums 21 eines synthetischen Sinussignals 20 und Auslösung eines Ereignistriggers 26. Dabei zeigt die X-Achse die Zeit in ms und die Y-Achse die Amplitude des Signals in mV. Das Sinussignal 20 ist ein durch die Anordnung gemessenes Signal. Dieses Signal 20 weist bei einer Phase von 90° ein Maximum 21 auf. Zur Analyse und Auslösung des Triggers wird das Sinussignal 20 zunächst in ein gefiltertes Signal 22 umgewandelt. Das gefilterte Signal 22 läuft dem ursprünglichen Signal 20 dabei um 1-2ms nach, was auf die Bearbeitungsdauer und Umwandlung des Originalsignals 20 das gefilterte Signal 22 in der Messvorrichtung zurückzuführen ist.

Das gefilterte Signal 22 weist entsprechend ein Maximum 23 auf, das um dieselbe Zeit von 1-2ms zu dem eigentlichen Maximum 21 des Messsignals 20 verzögert auftritt. Diese Zeitverzögerung entspricht bei der gezeigten Frequenz von ca. 10Hz in etwa einer Phase von 5°. Bei Erreichen des so bestimmten Maximums 23 wird zu diesem Zeitpunkt 27 ein Triggersignal 26 ausgelöst. Dieses Triggersignal 26 beträgt in der gezeigten Figur 5 100mV. Es versteht sich, dass bei tatsächlichen Messungen von Gewebe die Spannungswerte entsprechend einstellbar sind und beispielsweise der Messung bzw. Stimulation eines Gewebes dienen können. Die in Fig. 5 gezeigte Kurve 24 ist dabei die Phase des Signals.

Figur 6 zeigt die Detektion eines Amplitudenmaximums 33 eines tatsächlichen EEG-Signals 30, in der Figurenlegende mit online-re-referenced Pz bezeichnet, mit ausreichendem Signal-Störabstand. Die Kurve 32 stellt ein Sinussignal dar, das basierend auf einer offline - also gegebenenfalls nicht Echtzeit-Bedingungen erfüllenden - Signalfilterung errechnet wurde. Um Echtzeitbedingungen zu erfüllen, erfolgt durch eine online-Verarbeitung des Messsignals 30, basierend auf einem Görtzel-Algorithmus, die Erzeugung eines Phasensignals 34. Das Phasensignal 34, in der Legende auch als online-görtzel phase bezeichnet, verläuft hier stets linear von der Phase 0° bis 360°. Aus diesem Verlauf kann die Phase 90° bestimmt werden und der Triggerimpuls wird zu diesem Zeitpunkt 37 ausgelöst, zu erkennen an dem Triggersignal 36 in Figur 6. Die Auslösung des Ereignistriggers kann dabei erfindungsgemäß nach 1 bis 3 ms nach Erreichen des tatsächlichen Maximums des Messsignals 30 erfolgen.

Aufgrund der Anwendung in der Echtzeitumgebung ist das Messergebnis auf diese Weise schnell gemessen und ausgewertet, so dass für die Stimulation keine Vorhersage in die Zukunft durch den Algorithmus notwendig ist. Mit einem einfachen Görtzel-Algorithmus wird der Spektralanteil im a-EEG-Band (8 bis 12 Hz) zerlegt, die Phase bereits innerhalb von zwei Zyklen berechnet und das Ereignis (hier: Maximalamplitude, Phase 90°) detektiert

Durch den sehr geringen Jitter der Paketintervalle ist es möglich, Filterdurchlaufzeiten in die Berechnung mit einzubinden und so noch kleinere konstante Abweichungen zu erzeugen.

Die vorliegend exemplarisch gezeigten Kurven wurden dabei erstellt, ohne auf die üblichen Methoden zur Prädiktion des Signalverlaufes zurückzugreifen. Signal-Prädiktion ist dabei ein bisher übliches Mittel, um die Triggerimpulse in den richtigen Phasen auszulösen. Durch die erfindungsgemäße Echtzeit-Methode wird dabei eine größere Genauigkeit erlaubt, als dies eine Prädiktion erlaubt. Es ist jedoch denkbar, dass auch für die erfindungsgemäß ermöglichten Verfahren ebenfalls noch eine Prädiktion erfolgt.

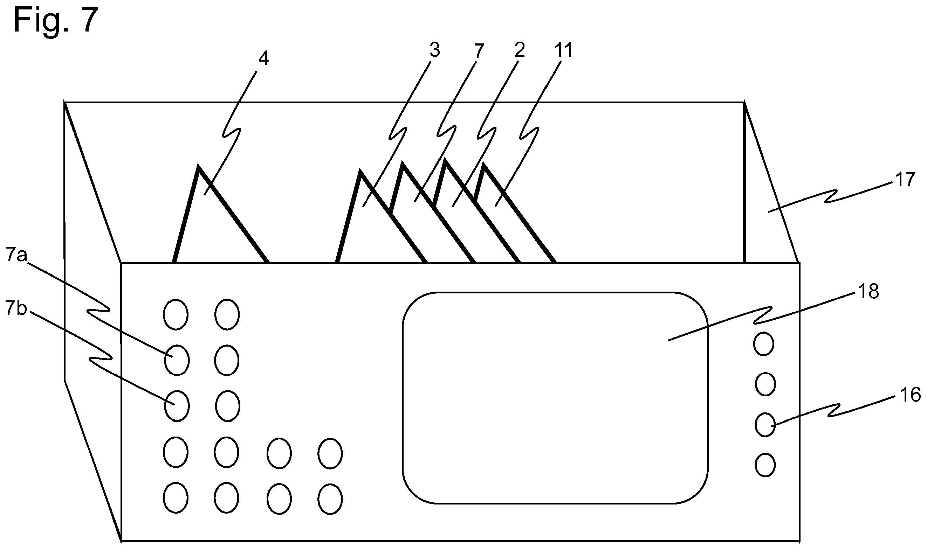

Fig. 7 zeigt ein Beispiel für eine Vorrichtung mit integriertem Stimulations- und Messsystem. Dabei weist die Vorrichtung ein Gehäuse 17 auf, in dem ein Mastermodul 4 sowie eine Mehrzahl an Funktionsmodulen 10, hier ein Erfassungsmodul 3, eine Steuerungsmodul 7 und ein Aktionsmodul 2 sowie ein Anzeigemodul 10, beispielsweise ein TFT Modul, auf. Die Funktionsmodule sind mit einer hier nicht sichtbaren BUS-Struktur verbunden. An einer Vorderseite des Gehäuses 17 ist eine Anzeigevorrichtung 18 ausgebildet. Die Anzeigevorrichtung 18 ist dabei mit dem Anzeigemodul 11 verbunden. Dabei kann die Anzeigevorrichtung 18 diverse Funktionalitäten aufweisen, wie zuvor beschrieben.

Zudem sind an der Vorderseite in der gezeigten Ausführungsform eine Reihe von Anschlüssen 16 vorgesehen. Einige der Anschlüsse sind dabei Dateneingänge 7a und/oder Datenausgänge 7b des Steuerungsmoduls 7 sein. Andere der Anschlüsse 16 sind Anschlüsse für andere der verwendeten Funktionsmodule oder für andere Funktionen in der Vorrichtung.

### Abkürzungen

- EKG -: Elektrokardiogramm
- EEG -: Elektroenzephalogramm
- EMG -: Elektromyogramm
- EOG -: Elektrookulogramm
- ERG -: Elektroretinogramm
- PPT -: Photoplethysmographie
- MKG -: Magnetkardiogramm
- MEG -: Magnetenzephalogramm
- BD/BP -: Blutdruck/Bloodpressure
- SpO2 -: Sauerstoffsättigung
- RT-BUS -: Real-Time Bus /Echtzeitbus
- USB -: Universal Series Bus
- LAN -: Local Area Network
- FES -: Funktionelle Elektrostimulation
- OS -: Operating System / Betriebssystem
- MIS -: Integriertes Stimulations- und Messsystem
- TMS -: Transkranielle Magnetstimulation
- tES -: transkranielle elektrische Stimulation
- nTMS -: navigierte transkranielle Magnetstimulation
- tDCS -: transcranial direct current stimulation - Gleichstromstimulation
- tACS -: transkranielle Wechselstromsimulation
- tRNS -: transcranial random noise stimulation - Rauschstimulation
- DBS -: deep brain stimulation
- iAPF -: EEG-Alpha-Peak-Frequenz
- FES -: phasenbezogene elektrische periphere Stimulation

### Bezugszeichen

- 1: System zur körperzustandsabhängigen Stimulation
- 2: Aktionsmodul / Current-Modul
- 3: Erfassungsmodul / EEG-Modul
- 4: Signalverarbeitungsmodul/Mastermodul
- 5: Kommunikationsverbindung
- 6: galvanische Trennung
- 7: Steuerungsmodul
- 7a: Dateneingang
- 7b: Datenausgang
- 9: Modulsteckplatz
- 10: Funktionsmodul
- 11: Anzeigemodul, TFT-Modul
- 12: Steuerabschnitt, RT-BUS Interface
- 13: Netzwerkmodul, LAN-Modul
- 14: Komponenten, externe Geräte
- 15: Kommunikationsmodul, COM-Modul
- 16: Anschlüsse
- 17: Gehäuse
- 18: Anzeige
- 20: Messsignal
- 21: Maximum des Messsignals
- 22: gefiltertes Signal
- 23: Maximum des gefilterten Signals
- 24: Phasensignal
- 26: Triggersignal
- 27: Triggerzeitpunkt
- 30: Messsignal
- 31: Maximum des Messsignals
- 32: gefiltertes Signal
- 33: Maximum des gefilterten Signals
- 34: Phasensignal
- 36: Triggersignal
- 37: Triggerzeitpunkt

## Patentansprüche

1. System zur körperzustandsabhängigen Stimulation aufweisend ein Mastermodul (4, Master) zur Verarbeitung von Modul-Signalen/Daten, und Funktionsmodule, wobei die Funktionsmodule
ein Aktionsmodul (2) zur Stimulation von Gewebe und ein Erfassungsmodul (3) zur Ableitung/Messung von Biodaten oder Biosignalen und
ein Steuerungsmodul zur Auslösung eines Triggerimpuls zur Steuerung des Aktionsmoduls umfassen,
wobei das System so ausgebildet ist, dass eine Kommunikation zwischen den Funktionsmodulen über eine Kommunikationsverbindung (5) erfolgt, die harte oder zumindest feste Echtzeitanforderungen erfüllt,
wobei das Erfassungsmodul zur Ableitung bzw. Messung eines EEG und das Aktionsmodul für eine transkranielle Stimulation von Gewebe ausgebildet sind,
wobei die Kommunikationsverbindung (5) einen echtzeitfähigen Bus umfasst, an den die Funktionsmodule angeschlossen sind,
**dadurch gekennzeichnet, dass**
das System eingerichtet ist, den Triggerimpuls zu einem Zeitpunkt einer vorbestimmten Phase des EEGs auszulösen, wobei unter Verwendung eines Görtzel-Algorithmus eine Zerlegung des EEG in ein Band von 8 bis 12 Hz erfolgt und innerhalb von zwei Zyklen des EEGs die vorbestimmte Phase des EEGs bestimmt wird.

2. System zur körperzustandsabhängigen Stimulation gemäß Anspruch 1, **dadurch gekennzeichnet, dass** an den echtzeitfähigen Bus das Steuerungsmodul (7) zur digitalen Input/Output-Steuerung von Aktoren zur Stimulation von Gewebe externer Geräte, angeschlossen ist.

3. System zur körperzustandsabhängigen Stimulation gemäß Anspruch 1 oder 2, **dadurch gekennzeichnet, dass** die Funktionsmodule (2, 3, 4, 7) dazu ausgelegt sind, dass sie auf dem echtzeitfähigen Bus (ECAT) Information im selben Berechnungsschritt in einem vom Mastermodul (4) abhängig von dessen zur Datenverarbeitung bereitgestellten Takt untereinander austauschen.

4. System zur körperzustandsabhängigen Stimulation gemäß einem der Ansprüche 1 bis 3, **dadurch gekennzeichnet, dass** das
Erfassungsmodul (3)
ausgebildet ist, gemessenen Daten/Signale über die Kommunikationsverbindung (5) zum Mastermodul (4) zu senden, das Mastermodul (4) ausgebildet ist, die empfangenen Daten zu verarbeiten, und das Mastermodul (4) weiterhin ausgebildet ist, Daten/Befehle an das Aktionsmodul (2) zu senden, um über Aktoren die Stimulation von Gewebe zu aktivieren.

5. System zur körperzustandsabhängigen Stimulation gemäß einem
der Ansprüche 1 bis 4, **dadurch gekennzeichnet,**
**dass** das Erfassungsmodul (3) einen A/D-Wandler zur Wandlung von durch dieses Modul erfassten analogen Biodaten/Biosignale in Digitalsignale umfasst, die im Mastermodul (4) verarbeitbar sind.

6. System zur körperzustandsabhängigen Stimulation gemäß einem der Ansprüche 1 bis 5, **dadurch gekennzeichnet, dass** das Aktionsmodul (2) einen D/A-Wandler zur Wandlung von vom Mastermodul (4) bereitgestellten digitalen Steuerungssignalen in Analogsignale umfasst, die Aktoren zur Stimulation von Gewebe zugeführt werden.

7. System zur körperzustandsabhängigen Stimulation gemäß einem der Ansprüche 1 bis 6, **dadurch gekennzeichnet, dass** das Steuerungsmodul (7) eingerichtet ist, eine **digitale** Input/Output-Steuerung des Signalflusses der von einem D/A-Wandler des Aktionsmoduls (2) und vom Mastermodul (4) bereitgestellten digitalen Steuerungssignale mittels vom Mastermodul (4) erzeugter und vom Steuerungsmodul erzeugter Triggersignale (26, 36) durchzuführen.

8. System zur körperzustandsabhängigen Stimulation gemäß einem der Ansprüche 1 bis 7, **dadurch gekennzeichnet, dass** eine galvanische Trennung (6) zwischen wenigstens einem Modul (4,7) und dem echtzeitfähigen Bus vorgesehen ist.

9. System zur körperzustandsabhängigen Stimulation gemäß einem der Ansprüche 1 bis 8 **dadurch gekennzeichnet, dass** der echtzeitfähige Bus ein EtherCAT-Bus ist.

## Claims

1. System for body-state-dependent stimulation, comprising a master module (4, Master) for processing module signals/data, and functional modules, wherein the functional modules include an action module (2) for stimulating tissue and a detection module (3) for deriving/measuring biodata or biosignals and a control module for triggering a trigger pulse for controlling the action module,
wherein the system is designed such that communication between the functional modules takes place via a communication link (5) which meets hard or at least firm real-time requirements,
wherein the detection module is designed for deriving or measuring an EEG and the action module is designed for transcranial tissue stimulation, wherein the communication link (5) comprises a real-time-capable bus to which the functional modules are connected,
**characterized in that**
the system is designed to trigger the trigger pulse at a point in time during a predetermined phase of the EEG, wherein a Goertzel algorithm is used to break down the EEG into a band of 8 to 12 Hz and, within two cycles of the EEG, to determine the predetermined phase of the EEG.

2. System for body-state-dependent stimulation according to claim 1,
**characterized in that** the control module (7) for digital input/output control of actuators of external devices, which actuators are intended for tissue stimulation, is connected to the real-time-capable bus.

3. System for body-state-dependent stimulation according to claim 1 or claim 2, **characterized in that** the functional modules (2, 3, 4, 7) are designed to exchange information with one another on the real-time-capable bus (ECAT) in the same calculation step in a clock cycle provided by the master module (4) depending on the data processing thereof.

4. System for body-state-dependent stimulation according to any of claims 1 to 3, **characterized in that** the detection module (3) is designed to send measured data/signals via the communication link (5) to the master module (4), the master module (4) is designed to process the received data, and the master module (4) is further designed to send data/commands to the action module (2) to activate tissue stimulation by means of actuators.

5. System for body-state-dependent stimulation according to any of claims 1 to 4, **characterized in that** the detection module (3) comprises an A/D converter for converting analog biodata/biosignals detected by this module into digital signals which can be processed in the master module (4).

6. System for body-state-dependent stimulation according to any of claims 1 to 5, **characterized in that** the action module (2) comprises a D/A converter for converting digital control signals provided by the master module (4) into analog signals which are supplied to actuators for tissue stimulation.

7. System for body-state-dependent stimulation according to any of claims 1 to 6, **characterized in that** the control module (7) is designed to carry out, by means of trigger signals (26, 36) generated by the master module (4) and by the control module, digital input/output control of the signal flow of the digital control signals provided by a D/A converter of the action module (2) and by the master module (4).

8. System for body-state-dependent stimulation according to any of claims 1 to 7, **characterized in that** galvanic isolation (6) is provided between at least one module (4,7) and the real-time-capable bus.

9. System for body-state-dependent stimulation according to any of claims 1 to 8, **characterized in that** the real-time-capable bus is an EtherCAT bus.

## Revendications

1. Système pour la stimulation en fonction de l'état du corps, présentant un module maître (4, Master) pour le traitement de signaux/données de module, et des modules fonctionnels, dans lequel les modules fonctionnels comprennent un module d'action (2) pour la stimulation de tissus et un module d'acquisition (3) pour la dérivation/mesure de biodonnées ou de biosignaux et un module de commande pour le déclenchement d'une impulsion de déclenchement pour la commande du module d'action,
dans lequel le système est configuré de sorte qu'une communication entre les modules fonctionnels est effectuée par l'intermédiaire d'une connexion de communication (5) qui satisfait à des exigences de temps réel sévères ou au moins fixes,
dans lequel le module d'acquisition est configuré pour la dérivation ou la mesure d'un EEG et le module d'action est configuré pour une stimulation transcrânienne de tissus, dans lequel la connexion de communication (5) comprend un bus capable de fonctionner en temps réel, auquel les modules fonctionnels sont raccordés,
**caractérisé en ce que**
le système est configuré pour déclencher l'impulsion de déclenchement à un moment d'une phase prédéterminée de l'EEG, dans lequel, à l'aide d'un algorithme de Goertzel, une décomposition de l'EEG en une bande allant de 8 à 12 Hz est effectuée et la phase prédéterminée de l'EEG est déterminée au cours de deux cycles de l'EEG.

2. Système pour la stimulation en fonction de l'état du corps selon la revendication 1, **caractérisé en ce que** le module de commande (7) pour la commande numérique d'entrée/de sortie d'actionneurs pour la stimulation de tissus d'appareils externes est raccordé au bus capable de fonctionner en temps réel.

3. Système pour la stimulation en fonction de l'état du corps selon la revendication 1 ou 2, **caractérisé en ce que** les modules fonctionnels (2, 3, 4, 7) sont adaptés pour échanger entre eux, sur le bus capable de fonctionner temps réel (ECAT), des informations au cours de la même étape de calcul, à une cadence fournie par le module maître (4) en fonction de son traitement de données.

4. Système pour la stimulation en fonction de l'état du corps selon l'une des revendications 1 à 3, **caractérisé en ce que** le module d'acquisition (3)
est configuré pour envoyer des données/signaux mesurés au module maître (4) par l'intermédiaire de la connexion de communication (5), le module maître (4) est configuré pour traiter les données reçues, et le module maître (4) est en outre configuré pour envoyer des données/instructions au module d'action (2) afin d'activer la stimulation de tissus par l'intermédiaire d'actionneurs.

5. Système pour la stimulation en fonction de l'état du corps selon l'une des revendications 1 à 4, **caractérisé en ce que** le module d'acquisition (3) comprend un convertisseur A/N pour la conversion de biodonnées/biosignaux analogiques acquis par ledit module en signaux numériques qui peuvent être traités dans le module maître (4).

6. Système pour la stimulation en fonction de l'état du corps selon l'une des revendications 1 à 5, **caractérisé en ce que** le module d'action (2) comprend un convertisseur N/A pour la conversion de signaux de commande numériques fournis par le module maître (4) en signaux analogiques qui sont amenés à des actionneurs pour la stimulation de tissus.

7. Système pour la stimulation en fonction de l'état du corps selon l'une des revendications 1 à 6, **caractérisé en ce que** le module de commande (7) est configuré pour réaliser une commande numérique d'entrée/de sortie du flux de signaux des signaux de commande numériques fournis par un convertisseur N/A du module d'action (2) et par le module maître (4) au moyen de signaux de déclenchement (26, 36) générés par le module maître (4) et générés par le module de commande.

8. Système pour la stimulation en fonction de l'état du corps selon l'une des revendications 1 à 7, **caractérisé en ce qu'un** moyen de séparation galvanique (6) est prévu entre au moins un module (4, 7) et le bus capable de fonctionner en temps réel.

9. Système pour la stimulation en fonction de l'état du corps selon l'une des revendications 1 à 8, **caractérisé en ce que** le bus capable de fonctionner en temps réel est un bus de type EtherCAT.
